(19) Europäisches Patentamt
European Patent Office
Office européen des brevets

(11) Veröffentlichungsnummer: **0 054 132**
**B1**

(12) # EUROPÄISCHE PATENTSCHRIFT

(45) Veröffentlichungstag der Patentschrift: **10.10.84**

(21) Anmeldenummer: **81108623.0**

(22) Anmeldetag: **21.10.81**

(51) Int. Cl.³: **C 07 D 239/91**, C 07 D 471/04, A 61 K 31/505 // (C07D471/04, 239/00, 221/00)

(54) **Neue Pyrimidinone, ihre Herstellung und Arzneimittel mit einem Gehalt an diesen Stoffen.**

(30) Priorität: **12.12.80 DE 3046871**
**16.04.81 DE 3115447**

(43) Veröffentlichungstag der Anmeldung:
**23.06.82 Patentblatt 82/25**

(45) Bekanntmachung des Hinweises auf die Patenterteilung:
**10.10.84 Patentblatt 84/41**

(84) Benannte Vertragsstaaten:
**AT BE CH DE FR IT LI LU NL SE**

(56) Entgegenhaltungen:
**DE - A - 1 939 109**
**US - A - 3 265 697**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Heider, Joachim, Dr. Dipl.-Chem., Am Hang 3, D-7951 Warthausen 1 (DE)**
Erfinder: **Austel, Volkhard, Dr. Dipl.-Chem., Kapellenweg 7, D-7950 Biberach 1 (DE)**
Erfinder: **Eberlein, Wolfgang, Dr. Dipl.-Chem., Obere Au 6, D-7950 Biberach 1 (DE)**
Erfinder: **Kadatz, Rudolf, Prof. Dr., Am Wolfgangsberg 22, D-7950 Biberach 1 (DE)**
Erfinder: **Lillie, Christian, Dr., Hansi Niese Weg 12, A-1130 Wien (AT)**

**EP 0 054 132 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

In der US-A-3 265 697 werden 2,3-Dihydro-4(1 H)-chinazolin-4-one, welche eine das Zentralnervensystem stimulierende Wirkung aufweisen, und in der DE-A-1 939 109 die Verbindung 2-Methyl-3-[2-(2-diäthylaminoäthoxy)-phenyl]chinazolin-4-on, welche eine ödemhemmende Wirkung aufweisen, beschrieben.

Es wurde nun gefunden, daß die neuen Pyrimidinone der allgemeinen Formel

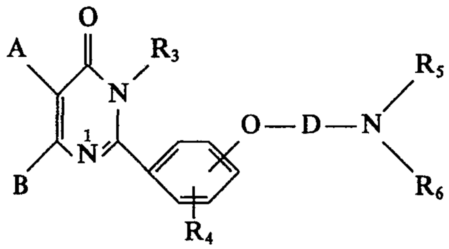

(I)

und deren Säureadditionssalze, insbesondere deren physiologisch verträgliche Säureadditionssalze mit anorganischen und organischen Säuren andere wertvolle pharmakologische Eigenschaften aufweisen, insbesondere eine blutdrucksenkende, antiarrhythmische und $\beta$- Rezeptoren-blockierende Wirkung.

Gegenstand der vorliegenden Erfindung sind somit die obigen neuen Verbindungen, Verfahren zu ihrer Herstellung und diese Verbindungen enthaltende Arzneimittel.

In der obigen allgemeinen Formel I bedeutet

A und B zusammen mit den beiden dazwischenliegenden Kohlenstoffatomen einen Pyridinring oder einen Phenylring, der durch die Reste $R_1$ und/oder $R_2$ substituiert sein kann, wobei

$R_1$ ein Halogenatom, eine Amino- oder Nitrogruppe, eine Alkyl- oder Alkoxygruppe mit jeweils 1 bis 3 Kohlenstoffatomen und

$R_2$ eine Alkoxygruppe mit 1 bis 3 Kohlenstoffatomen darstellen,

D eine gegebenenfalls durch eine Hydroxygruppe substituierte Alkylengruppe mit 3 oder 4 Kohlenstoffatomen,

$R_3$ und $R_5$, die gleich oder verschieden sein können, je ein Wasserstoffatom oder eine Alkylgruppe mit 1 bis 3 Kohlenstoffatomen,

$R_4$ ein Wasserstoffatom oder eine Alkoxygruppe mit 1 bis 3 Kohlenstoffatomen und

$R_6$ eine geradkettige oder verzweigte Alkylgruppe mit 1 bis 6 Kohlenstoffatomen oder eine gegebenenfalls durch eine Hydroxygruppe substituierte geradkettige gesättigte Alkylengruppe mit 2 bis 4 Kohlenstoffatomen, welche endständig durch eine Phenyl- oder Phenoxygruppe substituiert ist, wobei der Phenylkern jeweils durch Alkyl- und/oder Alkoxygruppen mit jeweils 1 bis 3 Kohlenstoffatomen mono- oder disubstituiert sein kann.

Für die bei der Definition der Reste A, B, D, $R_3$, $R_4$, $R_5$ und $R_6$ eingangs erwähnten Bedeutungen kommen beispielsweise für

A und B zusammen mit den beiden dazwischenliegenden Kohlenstoffatomen die des Pyridin-, Phenyl-, Chlorphenyl-, Bromphenyl-, Fluorphenyl-, Aminophenyl-, Nitrophenyl-, Methoxyphenyl-, Äthoxyphenyl-, Propoxyphenyl-, Isopropoxyphenyl-, Dimethoxyphenyl-, Diäthoxyphenyl-, Methoxy-äthoxyphenyl-, Methoxy-propoxyphenyl-, Äthoxy-isopropoxyphenyl-, Methoxy-chlorphenyl-, Äthoxy-bromphenyl- oder Isopropoxy-fluorphenylringes, für

D die der n-Propylen-, n-Butylen-, 2-Hydroxy-n-propylen-, 2-Hydroxy-n-butylen- oder 3-Hydroxy-n-butylengruppe, für

$R_3$ und $R_5$ jeweils die eines Wasserstoffatoms, der Methyl-, Äthyl-, Propyl- oder Isopropylgruppe, für

$R_4$ die eines Wasserstoffatoms, der Methoxy-, Äthoxy-, Propoxy- oder Isopropoxygruppe, für

$R_6$ die der Methyl-, Äthyl-, Propyl-, Isopropyl-, Butyl-, Isobutyl-, tert.Butyl-, Pentyl-, Neopentyl-, tert.-Pentyl-, Hexyl-, 2-Phenyläthyl-, 3-Phenylpropyl-, 4-Phenylbutyl-, 2-Hydroxy-3-phenylpropyl-, 2-Hydroxy-4-phenylbutyl-, 3-Hydroxy-4-phenyl-butyl-, 2-(Methoxyphenyl)-äthyl-, 3-(Methoxyphenyl)-propyl-, 4-(Methoxyphenyl)-butyl-, 2-Hydroxy-3-(methoxyphenyl)-propyl-, 2-(Äthoxyphenyl)-äthyl-, 3-(Isopropoxyphenyl)-propyl-, 2-(Dimethoxyphenyl)-äthyl-, 3-(Dimethoxyphenyl)-propyl-, 2-Hydroxy-3-(dimethoxyphenyl)-propyl-, 2-(Methylphenyl)-äthyl-, 2-(Isopropylphenyl)-äthyl-, 3-(Methylphenyl)-propyl-, 2-Hydroxy-3-(methylphenyl)-propyl-, 4-(Methylphenyl)-butyl-, 2-(Dimethylphenyl)-äthyl-, 3-(Dimethylphenyl)-propyl-, 2-Hydroxy-3-(dimethylphenyl)-propyl-, 2-Phenoxyäthyl-, 3-Phenoxypropyl-, 4-Phenoxybutyl-, 2-Hydroxy-3-phenoxypropyl-,

2-Hydroxy-4-phenoxybutyl-, 3-Hydroxy-4-phenoxybutyl-, 2-(Methoxyphenoxy)-äthyl-, 3-(Methoxyphenoxy)-propyl-, 4-(Methoxyphenoxy)-butyl-, 2-(Äthoxyphenoxy)-äthyl-, 3-(Isopropoxyphenoxy)-propyl-, 2-(Dimethoxyphenoxy)-äthyl-, 3-(Dimethoxyphenoxy)-propyl-, 2-Hydroxy-3-(dimethoxyphenoxy)-propyl-, 2-Hydroxy-4-(dimethoxyphenoxy)-butyl-, 2-(Methylphenoxy)-äthyl-, 2-(Isopropylphenoxy)-äthyl-, 3-(Methylphenoxy)-propyl-, 2-Hydroxy-3-(methylphenoxy)-propyl-, 4-(Methylphenoxy)-butyl-, 2-(Dimethylphenoxy)-äthyl-, 3-(Dimethylphenoxy)-propyl-, 2-Hydroxy-3-(dimethylphenoxy)-propyl-, 2-(Methyl-methoxyphenyl)-äthyl-, 3-(Methyl-methoxyphenyl)-propyl-, 2-Hydroxy-3-(methyl-methoxyphenyl)-propyl-, 4-(Methyl-methoxyphenyl)-butyl-, 2-(Methyl-äthoxyphenyl)-äthyl-, 3-(Äthyläthoxyphenyl)-propyl-, 2-Hydroxy-3-(methylpropoxyphenyl)-propyl-, 2-(Methyl-methoxyphenoxy)-äthyl-, 3-(Methyl-methoxyphenoxy)-propyl-, 2-Hydroxy-3-(methyl-methoxyphenoxy)-propyl-, 4-(Methyl-methoxyphenoxy)-butyl-, 2-(Isopropyl-methoxyphenoxy)-äthyl-, 2-Hydroxy-3-(methyl-isopropoxyphenoxy)-propyl- oder 2-Hydroxy-3-(isopropyl-isopropoxyphenoxy)-propylgruppe in Betracht.

Bevorzugte Verbindungen der obigen allgemeinen Formel I sind jedoch diejenigen, in denen

A und B zusammen mit den beiden dazwischenliegenden Kohlenstoffatomen, einen Pyridinring oder einen Phenylring, der durch die Reste $R_1$ und/oder $R_2$ substituiert sein kann, wobei $R_1$ ein Chloratom, eine Methyl-, Methoxy- oder Nitrogruppe und
$R_2$ eine Methoxygruppe darstellen,
D eine n-Propylen-, n-Butylen-, 2-Hydroxy-n-propylen-, 2-Hydroxy-n-butylen- oder 3-Hydroxy-n-butylengruppe,
$R_3$ und $R_5$, die gleich oder verschieden sein können, je eine Alkylgruppe mit 1 bis 3 Kohlenstoffatomen oder ein Wasserstoffatom,
$R_4$ ein Wasserstoffatom oder die Methoxygruppe und
$R_6$ eine Alkylgruppe mit 1 bis 4 Kohlenstoffatomen, eine in 2-Stellung durch eine Methoxyphenyl-, Dimethoxyphenyl-, Methylphenoxy- oder Methoxyphenoxygruppe substituierte Äthylgruppe oder eine in 3-Stellung durch eine Methoxyphenoxy- oder Methylphenoxygruppe substituierte 2-Hydroxypropylgruppe bedeuten, und deren physiologisch verträgliche Säureadditionssalze.

Besonders bevorzugte Verbindungen der obigen allgemeinen Formel I sind jedoch diejenigen, in denen

A und B zusammen mit den beiden dazwischenliegenden Kohlenstoffatomen einen Phenyl-, Methoxyphenyl-, Dimethoxyphenyl- oder Pyridinring,
D die n-Propylen- oder 2-Hydroxy-n-propylengruppe,
$R_3$ ein Wasserstoffatom oder die Methylgruppe,
$R_4$ ein Wasserstoffatom oder die Methoxygruppe,
$R_5$ ein Wasserstoffatom oder eine Alkylgruppe mit 1 bis 3 Kohlenstoffatomen und
$R_6$ eine Alkylgruppe mit 1 bis 4 Kohlenstoffatomen, eine in 2-Stellung durch eine Methoxyphenyl-, Dimethoxyphenyl-, Methylphenoxy- oder Methoxyphenoxygruppe substituierte Äthylgruppe oder eine in 3-Stellung durch eine Methoxyphenoxy- oder Methylphenoxygruppe substituierte 2-Hydroxypropylgruppe bedeuten, insbesondere die Verbindungen der allgemeinen Formel

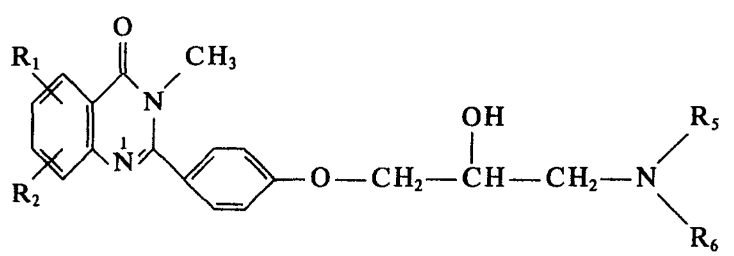

(Ia)

in der

$R_1$ in 6- oder 8-Stellung eine Methoxygruppe,
$R_2$ ein Wasserstoffatom oder in 7-Stellung eine Methoxygruppe und
$R_5$ und $R_6$ zusammen mit dem dazwischenliegenden Stickstoffatom eine Isopropylamino-, tert.Butylamino-, N-Methyl-2-(3,4-dimethoxy-phenyl)-äthylamino-, 2-(2-Methoxyphenyl)-äthylamino-, 2-(2-Methylphenoxy)-äthylamino- oder 2-(4-Methoxyphenoxy)-äthylaminogruppe bedeuten, und deren physiologisch verträgliche Säureadditionssalze mit anorganischen oder organischen Säuren.

Erfindungsgemäß erhält man die neuen Verbindungen nach folgenden Verfahren:

a) Durch Umsetzung eines Propoxyphenylderivates der allgemeinen Formel

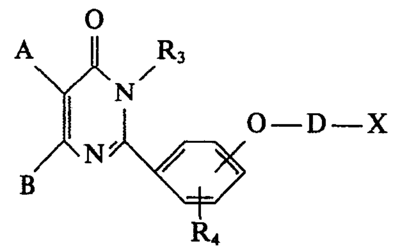

(II)

in der

$R_3$, $R_4$, A, B und D wie eingangs definiert sind,
X eine nukleophil austauschbare Gruppe wie ein Halogenatom oder
X zusammen mit einem β-ständigen Wasserstoffatom des Restes
D ein Sauerstoffatom darstellt, mit einem Amin der allgemeinen Formel

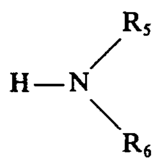

(III)

in der

$R_5$ und $R_6$ wie eingangs definiert sind.

Die Umsetzung erfolgt gegebenenfalls in einem Lösungsmittel, z. B. in Äthanol, Isopropanol, Tetrahydrofuran, Toluol, Dimethylformamid oder Dimethylsulfoxid, gegebenenfalls in Gegenwart eines säurebindenden Mittels, z. B. eines Alkoholats oder Alkalicarbonats wie Kalium-tert.butylat oder Kaliumkarbonat, und gegebenenfalls in einem Druckgefäß bei Temperaturen zwischen 50 und 200°C, vorzugsweise jedoch bei Temperaturen zwischen 70 und 150°C. Besonders vorteilhaft wird die Umsetzung jedoch unter Verwendung eines Überschusses des eingesetzten Amins der allgemeinen Formel III als Lösungsmittel durchgeführt.

b) Alkylierung einer Verbindung der allgemeinen Formel

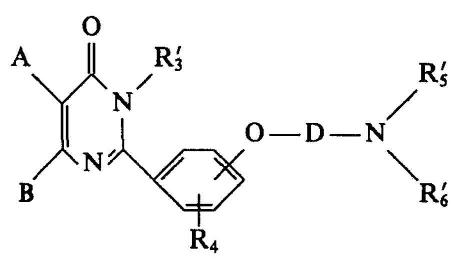

(IV)

in der

$R_4$, A, B und D wie eingangs definiert sind,
mindestens einer der Reste $R_3'$, $R_5'$ oder $R_6'$ ein Wasserstoffatom darstellt und die übrigen der Reste $R_3'$, $R_5'$ oder
$R_6'$ die für
$R_3$, $R_5$ oder $R_6$ eingangs erwähnten Bedeutungen besitzen, mit einer Verbindung der allgemeinen Formel

$$Z—Y \quad (V)$$

in der

Z eine nukleophil austauschbare Gruppe wie ein Halogenatom oder eine Sulfonyloxygruppe, z. B. ein Chlor-, Brom- oder Jodatom, eine Methylsulfonyloxy-, p-Toluolsulfonyloxy- oder Methoxysulfonyloxygruppe, oder Z zusammen mit einem β-ständigen Wasserstoffatom des Restes $R_6$ ein Sauerstoffatom darstellt und
Y mit Ausnahme der eines Wasserstoffatoms die für $R_3$, $R_5$ und $R_6$ eingangs erwähnten Bedeutungen besitzt, oder mit Formaldehyd/Ameisensäure.

Die Alkylierung wird vorzugsweise in einem Lösungsmittel wie Aceton, Tetrahydrofuran, Dioxan, Dimethylformamid oder Dimethylsulfoxyd mit einem entsprechenden Alkylhalogenid, z. B. mit Methyljodid, Äthylbromid, tert.Butylchlorid, 2-(2-Methoxyphenyl)-äthylbromid, 2-(2-Methylphenoxy)-äthylbromid oder 1-Chlor-2-hydroxy-3-(4-methoxyphenoxy)-propan, mit einer entsprechenden Sulfonyloxyverbindung, z. B. mit Dimethylsulfat, Diäthylsulfat oder tert.Butyl-p-toluolsulfonat oder einem entsprechenden Epoxid, z. B. mit 3-(4-Methoxyphenoxy)-propylenoxid, gegebenenfalls in Gegenwart einer anorganischen oder tertiären organischen Base, z. B. in Gegenwart von Kaliumkarbonat, Kalium-tert.butylat, Triäthylamin oder Pyridin, wobei die letzteren auch als Lösungsmittel dienen können, bei Temperaturen zwischen 0 und 180°C, vorzugsweise jedoch bei der Siedetemperatur des Reaktionsgemisches, durchgeführt.

Die Methylierung kann jedoch auch mit Formaldehyd/Ameisensäure bei erhöhten Temperaturen, vorzugsweise jedoch bei der Siedetemperatur des Reaktionsgemisches, durchgeführt werden.

Die erfindungsgemäß erhaltenen neuen Verbindungen lassen sich anschließend gewünschtenfalls in ihre Säureadditionssalze, insbesondere in ihre physiologisch verträglichen Salze mit anorganischen und organischen Säuren, überführen. Als Säuren kommen beispielsweise Salzsäure, Bromwasserstoffsäure, Schwefelsäure, Phosphorsäure, Milchsäure, Zitronensäure, Weinsäure, Oxalsäure oder Maleinsäure in Betracht.

Die als Ausgangsstoffe verwendeten Verbindungen der allgemeinen Formeln II und III erhält man nach literaturbekannten Verfahren bzw. sind literaturbekannt. So erhält man beispielsweise eine Verbindung der allgemeinen Formel II durch Umsetzung (siehe beispielsweise J. chem. Soc. 1972, 709; DE-OS 2 114 884 und Syn. Comm. 10, 241—243 [1980]) eines entsprechenden Oxazins der allgemeinen Formel

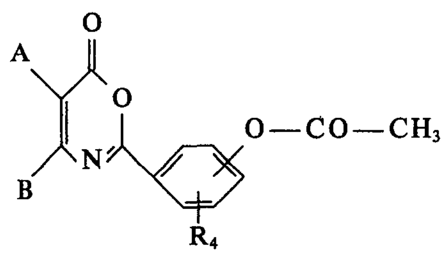

(VI)

mit einem entsprechenden Amin der allgemeinen Formel

$$R_3—NH_2 \qquad (VII)$$

anschließender Abspaltung des Acetylrestes und anschließender Alkylierung mit einem entsprechenden Halogenid der allgemeinen Formel

$$Br—D—X \qquad (VIII)$$

in denen

A, B, D, $R_3$, $R_4$ und X wie eingangs definiert sind.

Eine als Ausgangsstoff verwendete Verbindung der allgemeinen Formel IV erhält man beispielsweise durch Umsetzung eines entsprechenden Pyrimidin-4-ons mit einem entsprechenden Amin.

Wie bereits eingangs erwähnt, weisen die neuen Verbindungen der allgemeinen Formel I sowie deren physiologisch verträgliche Säureadditionssalze mit anorganischen und organischen Säuren wertvolle pharmakologische Eigenschaften auf, insbesondere eine blutdrucksenkende, antiarrhythmische und β-rezeptorenblockierende Wirkung.

Beispielsweise wurden die Verbindungen

A = 2-[4-(2-Hydroxy-3-tert.butylamino-propoxy)-phenyl]-3-methyl-6-methoxy-3,4-dihydrochinazolin-4-on,

B = 2-[4-[2-Hydroxy-3-(2-(3,4-dimethoxyphenyl)-N-methyl-äthylamino)-propoxy]-phenyl]-3-methyl-6-methoxy-3,4-dihydrochinazolin-4-on-dihydrochlorid,

C = 2-[4-[2-Hydroxy-3-(2-(2-methoxyphenyl)-äthylamino)-propoxy]-phenyl]-3-methyl-6-methoxy-3,4-dihydro-chinazolin-4-on-dihydrochlorid,

D = 2-[4-(2-Hydroxy-3-isopropylamino-propoxy)-phenyl]-3-methyl-6-methoxy-3,4-dihydrochinazolin-4-on,

E = 2-[4-[2-Hydroxy-3-(2-(2-methylphenoxy)-äthylamino)-propoxy]-phenyl]-3-methyl-6,7-dimethoxy-3,4-dihydro-chinazolin-4-on und

F = 2-[4-[3-(2-Hydroxy-3-(3-methylphenoxy)-propylamino)-propoxy]-phenyl]-3-methyl-8-methoxy-3,4-dihydro-chinazolin-4-on

auf ihre biologischen Eigenschaften hin untersucht:

## 1. Wirkung auf den Kreislauf

Die Kreislaufversuche wurden an mischrassigen Hunden beider Geschlechter mit einem Gewicht von 18—29 kg in Chloralose-Urethan-Nembutal-Narkose (54 + 270 + 10 mg/kg i. v.) durchgeführt. Die Tiere wurden hierbei nach Thoraxeröffnung im 4. linken Intercostalraum mit einem Harvard-Respirator mit Raumluft beatmet.

Der arterielle Blutdruck wurde in einer Arteria carotis mit einem Statham-Druckwandler, die Herzfrequenz elektronisch aus der Folge der R-Zacken des Elektrocardiogramms bestimmt. Die maximale Druckanstiegsgeschwindigkeit (dp/dt max) im linken Ventrikel wurde mit einem Konigsberg Druckaufnehmer und einem Grass-Differensierverstärker gemessen.

Alle Parameter wurden mittels eines Direktschreibers registriert. Zur Gerinnungshemmung erhielten die Tiere 10 mg/kg polyäthylensulfonsaures Natrium intravenös, gelöst in 20%igem Polydiol. Die zu untersuchenden Substanzen wurden an 3 bis 4 Hunden intravenös appliziert.

Die nachfolgende Tabelle enthält die gefundenen Werte:

| Substanz | Dosis, mg/kg i. v. | Beeinflussung d. mittl. art. Blutdruckes [mm Hg] | Senkung der Herzfrequenz [1/min] | Senkung der Kontraktilität, dp/dt max % | Dauer, min |
|---|---|---|---|---|---|
| A | 0,5 | −20/− 8 | −21 | −33 | >55 |
| B | 1,0 | −16/−24 | −28 | −17 | 45 |
| C | 1,0 | + 1/− 3 | −22 | −14 | >36 |
| D | 1,0 | − 5/− 2 | −30 | −27 | >40 |
| E | 1,0 | −13/−14 | −11 | −14 | >36 |
| F | 1,0 | −11/−18 | −23 | −23 | >63 |

## 2. Wirkung auf die Kontraktionskraft und die Frequenz des isolierten Meerschweinchenvorhofes

Die Versuche wurden durchgeführt an 4—5 isolierten spontan schlagenden Meerschweinchen-Vorhöfen pro Substanz in Tyrode-Lösung von 30°C mit Carbogen durchperlt. Die Spannungsregistrierung erfolgte mittels Dehnungsmeßstreifen isometrisch auf einem Grass-Polygraph. Die Substanzen wurden in steigenden Konzentrationen kumulativ in das Organbad gegeben, zwischen den einzelnen Konzentrationsschritten lagen jeweils 10 Minuten. Wegen der schlechten Wasserlöslichkeit wurde bei der Herstellung der Verdünnungen jeweils von 1%igen Lösungen in Polydiol ausgegangen, welche in Tyrode-Lösung weiterverdünnt wurden. Die dabei erreichten Endkonzentrationen von Polydiol im Organbad von 1 : 1000 sind pharmakologisch unwirksam.

Die nachfolgende Tabelle enthält die gefundenen Werte:

| Substanz | Kontraktilität, % | | | | Frequenz, % | | | |
|---|---|---|---|---|---|---|---|---|
| | $10^{-6}$ | $3.10^{-6}$ | $10^{-5}$ | $3.10^{-5}$ | $10^{-6}$ | $3.10^{-6}$ | $10^{-5}$ | $3.10^{-5}$ |
| A | −4,3 | −11,4 | −20,8 | −46,8 | −5,5 | − 6,2 | −14,0 | −32,7 |
| B | −4,7 | − 9,0 | −27,5 | −49,1 | −3,6 | −12,7 | −28,4 | −53,3 |
| C | −7,5 | −16,5 | −34,6 | −62,6 | −3,5 | −10,2 | −23,6 | −42,5 |

Fortsetzung

| Substanz | Kontraktilität, % | | | | Frequenz, % | | | |
|---|---|---|---|---|---|---|---|---|
| | $10^{-6}$ | $3.10^{-6}$ | $10^{-5}$ | $3.10^{-5}$ | $10^{-6}$ | $3.10^{-6}$ | $10^{-5}$ | $3.10^{-5}$ |
| D | −3,3 | − 8,7 | −18,0 | −35,4 | −4,6 | −10,6 | −24,6 | −42,4 |
| E | −3,2 | − 9,3 | −16,1 | −30,6 | −3,1 | − 6,6 | −17,8 | −28,5 |
| F | −3,0 | − 4,4 | − 9,4 | −27,3 | −4,1 | −10,5 | −23,3 | −38,6 |

3. Bestimmung der akuten Toxizitäten

Die akute Toxizität der zu untersuchenden Substanzen wurde an Mäusen (Beobachtungszeit: 14 Tage) nach intravenöser Gabe orientierend bestimmt.

| Substanz | Akute Toxizität |
|---|---|
| A | >35 mg/kg i. v. (0 von 5 Tieren gestorben) |
| B | >35 mg/kg i. v. (0 von 5 Tieren gestorben) |
| C | >35 mg/kg i. v. (0 von 5 Tieren gestorben) |
| E | >35 mg/kg i. v. (0 von 5 Tieren gestorben) |
| F | >35 mg/kg i. v. (0 von 5 Tieren gestorben) |

Aufgrund ihrer pharmakologischen Eigenschaften eignen sich die erfindungsgemäß hergestellten neuen Verbindungen und deren physiologisch verträglichen Säureadditionssalze somit insbesondere zur Behandlung von Coronarerkrankungen und des Hochdrucks. Zur pharmazeutischen Anwendung lassen sich diese hierzu, gegebenenfalls in Kombination mit anderen Wirksubstanzen, in die üblichen galenischen Zubereitungsformen wie Tabletten, Dragées, Kapseln, Pulver, Suspensionen, Ampullen, Tropfen oder Suppositorien einarbeiten. Die Einzeldosis beträgt hierbei am Erwachsenen bei intravenöser Applikation 20—50 mg und bei oraler Applikation 50—250 mg 2- bis 3mal täglich.
Die nachstehenden Beispiele dienen der näheren Erläuterung der Erfindung. Die chemische Struktur der neuen Verbindungen wurde mittels IR-, UV-, NMR-Spektren und Elementaranalyse sichergestellt.

Beispiel I

2-(4-Hydroxyphenyl)-3-methyl-6-methoxy-3,4-dihydro-chinazolin-4-on

a) 2-(4-Acetoxyphenyl)-6-methoxy-3,1-benzoxazin-4-on

16,7 g (0,1 Mol) 3-Methoxyanthranilsäure werden in 100 ml Pyridin gelöst und unter Kühlung und Rühren mit 23,8 g (0,1 Mol + 20 %) 4-Acetoxybenzoylchlorid versetzt. Nach 2 Stunden ist die Umsetzung beendet und das Reaktionsgemisch wird auf Eiswasser gegossen. Der dabei ausgefallene Niederschlag wird abgesaugt, getrocknet und aus Methanol kristallisiert.
Schmelzpunkt: 164—166°C,
Ausbeute: 20,2 g (65 % der Theorie)

$C_{17}H_{13}NO_5$ (311,28)

| | | | |
|---|---|---|---|
| Ber.: | C 65,59 | H 4,21 | N 4,50 |
| Gef.: | C 65,51 | H 4,27 | N 4,58 |

b) 2-(4-Hydroxyphenyl)-3-methyl-6-methoxy-3,4-dihydro-chinazolin-4-on

6,2 g (0,02 Mol) des gemäß Beispiel a) erhaltenen Produktes werden mit 60 ml 40 % Methylaminlösung in Wasser in einer Stahlbombe auf 120 °C erhitzt. Nach 3 Stunden ist die Umsetzung beendet, die Reaktionslösung wird im Vakuum eingedampft und das erhaltene Rohprodukt aus Methanol kristallisiert.
Schmelzpunkt: 146–147 °C,
Ausbeute: 4,8 g (85 % der Theorie)

$C_{16}H_{14}N_2O_3$ (282,30)

| | | | |
|---|---|---|---|
| Ber.: | C 68,08 | H 5,00 | N 9,92 |
| Gef.: | C 67,95 | H 5,05 | N 9,86 |

Beispiel II

2-(4-Hydroxyphenyl)-3,6-dimethyl-3,4-dihydro-chinazolin-4-on

a) 2-(4-Acetoxyphenyl)-6-methyl-3,1-benzoxazin-4-on

10 g (0,066 Mol) 5-Methylanthranilsäure werden in 65 ml Pyridin gelöst und unter Kühlen und Rühren mit 15,7 g (0,066 Mol + 20 %) 4-Acetoxybenzoylchlorid versetzt. Nach 2 Stunden ist die Umsetzung beendet und das Reaktionsgemisch wird auf Eiswasser gegossen. Der dabei ausgefallene Niederschlag wird abgesaugt, gut mit Wasser gewaschen und getrocknet.
Schmelzpunkt: 182–184 °C,
Ausbeute: 17,2 g (88 % der Theorie)

$C_{17}H_{13}NO_4$ (295,29)

| | | | |
|---|---|---|---|
| Ber.: | C 69,15 | H 4,44 | N 4,72 |
| Gef.: | C 69,15 | H 4,61 | N 4,72 |

b) 2-(4-Hydroxybenzoylamino)-5-methyl-benzoesäure-monomethyl-amid

10 g (0,034 Mol) des gemäß Beispiel a) erhaltenen Produktes werden in 100 ml 30%iger Methylaminlösung in Wasser auf dem Dampfbad erhitzt. Nach 30 Minuten wird die Lösung im Vakuum eingedampft und der dabei erhaltene weiße Rückstand getrocknet.
Schmelzpunkt: 250–252 °C,
Ausbeute: 9,3 g (97 % der Theorie)

$C_{16}H_{16}N_2O_3$ (284,32)

| | | | |
|---|---|---|---|
| Ber.: | C 67,59 | H 5,67 | N 9,85 |
| Gef.: | C 67,39 | H 5,88 | N 9,81 |

c) 2-(4-Hydroxyphenyl)-3,6-dimethyl-3,4-dihydro-chinazolin-4-on

5 g (0,018 Mol) des gemäß Beispiel b) erhaltenen Produktes werden in 40 ml Äthylenglycol und 0,5 ml N,N-Dimethylaminoäthanol auf 180 °C erwärmt. Nach einer Stunde ist die Umsetzung beendet, die Reaktionslösung wird abgekühlt und mit Eiswasser versetzt, wobei das Endprodukt auskristallisiert.
Schmelzpunkt: 228–230 °C,
Ausbeute: 4,2 g (89 % der Theorie)

$C_{16}H_{14}N_2O_2$ (266,30)

| | | | |
|---|---|---|---|
| Ber.: | C 72,17 | H 5,30 | N 10,50 |
| Gef.: | C 72,13 | H 5,27 | N 10,52 |

Beispiel III

2-(4-Hydroxyphenyl)-3-methyl-6-methoxy-3,4-dihydro-chinazolin-4-on

a) 2-(4-Acetoxyphenyl)-3-methyl-6-methoxy-3,4-dihydro-chinazolin-4-on

Eine auf –30 °C gekühlte Lösung von 55,5 g (0,332 Mol) 3-Methoxyanthranilsäure in 500 ml Pyridin

wird bei −30°C unter Rühren zu 91 g (0,425 Mol) 4-Acetoxyphenyl-methylimidchlorid gegeben. Das Reaktionsgemisch wird eine Stunde bei 0°C und eine weitere Stunde bei Raumtemperatur gerührt. Anschließend gießt man das dunkelviolett verfärbte Reaktionsgemisch auf ca. 2,8 l Eiswasser, wobei sich das gewünschte Produkt in violetten Kristallen abscheidet. Der Niederschlag wird abgesaugt, mit Wasser gewaschen und im Vakuumtrockenschrank bei 80°C getrocknet.
Schmelzpunkt: 164–170°C,
Ausbeute: 80,4 g (74,6% der Theorie)

$C_{18}H_{16}N_2O_4$ (324,33)

| | | | |
|---|---|---|---|
| Ber.: | C 66,65 | H 4,97 | N 8,64 |
| Gef.: | C 66,29 | H 4,76 | N 8,53 |

b) 2-(4-Hydroxyphenyl)-3-methyl-6-methoxy-3,4-dihydro-chinazolin-4-on

80,3 g (0,247 Mol) 2-(4-Acetoxyphenyl)-3-methyl-6-methoxy-3,4-dihydro-chinazolin-4-on werden in 800 ml konzentriertem wäßrigem Ammoniak suspendiert und auf dem Dampfbad 1½ Stunden erhitzt, wobei zunächst eine klare Lösung eintrat, und sich nach ca. 60 Minuten ein weißer Niederschlag abzuscheiden begann. Nach dem Abkühlen auf 0°C, wird das Produkt abgesaugt, mit Wasser gewaschen und im Umlufttrockenschrank bei 80°C getrocknet.
Schmelzpunkt: 263–268°C,
Ausbeute: 65,7 g (94% der Theorie)

$C_{16}H_{14}N_2O_3$ (282,29)

| | | | |
|---|---|---|---|
| Ber.: | C 68,07 | H 5,00 | N 9,92 |
| Gef.: | C 68,25 | H 5,16 | N 9,69 |

## Beispiel IV

### 2-(4-Hydroxy-phenyl)-3-methyl-3,4-dihydro-pyrido[2,3-e]pyrimidin-4-on

a) 2-(4-Acetoxy-phenyl)-pyrido[5,6-b]-3,1-oxazin-4-on

27,6 g (0,2 Mol) 2-Amino-nicotinsäure werden in 250 ml Pyridin suspendiert und unter Kühlen und Rühren mit 50,0 g (0,25 Mol) 4-Acetoxybenzoylchlorid versetzt. Nach 2 Stunden ist die Reaktion beendet und das Gemisch wird auf ca. 1,5 l Eiswasser gegossen, wobei das gewünschte Produkt ausfällt. Der Niederschlag wird abgesaugt, gut mit Wasser gewaschen und bei 60°C im Vakuumtrockenschrank getrocknet.
Schmelzpunkt: 193–196°C,
Ausbeute: 43,5 g (77% der Theorie)

$C_{15}H_{10}N_2O_4$ (282,25)

| | | | |
|---|---|---|---|
| Ber.: | C 63,82 | H 3,57 | N 9,92 |
| Gef.: | C 63,71 | H 3,51 | N 9,87 |

b) 2-(4-Hydroxy-benzamido)-nicotinsäure-methylamid

30 g (0,106 Mol) des gemäß Beispiel a) erhaltenen Produktes werden in 300 ml 30%iger Methylaminlösung in Wasser auf dem Dampfbad erhitzt. Nach 10 Minuten wird die Lösung im Vakuum eingedampft und der kristalline Rückstand mit ca. 300 ml Ethanol aufgekocht und abgesaugt. Der erhaltene Niederschlag wird bei 60°C im Vakuumtrockenschrank getrocknet.
Schmelzpunkt: 218–220°C,
Ausbeute: 19,8 g (73% der Theorie)

$C_{14}H_{13}N_3O_3$ (271,26)

| | | | |
|---|---|---|---|
| Ber.: | C 61,98 | H 4,83 | N 15,49 |
| Gef.: | C 61,87 | H 4,69 | N 15,52 |

c) 2-(4-Hydroxy-phenyl)-3-methyl-3,4-dihydro-pyrido[2,3-e]-pyrimidin-4-on

18,5 g (0,068 Mol) des gemäß Beispiel b) erhaltenen Produktes werden in 200 ml Äthylenglykol und 4 ml N,N-Dimethylaminoäthanol eine Stunde lang auf 180°C erwärmt. Anschließend wird die Reaktionslösung abgekühlt und auf 1,5 l Eiswasser gegossen. Das auskristallisierte Endprodukt wird abgesaugt, mit viel Wasser nachgewaschen und bei 60°C im Vakuumtrockenschrank getrocknet.
Schmelzpunkt: 256–258°C,
Ausbeute: 9,4 g (54,6 % der Theorie)

$C_{14}H_{11}N_3O_2$ (253,25)

| | | | |
|---|---|---|---|
| Ber.: | C 66,39 | H 4,38 | N 16,59 |
| Gef.: | C 66,43 | H 4,46 | N 16,62 |

Beispiel V

2-[4-(4-Aminobutoxy)-phenyl]-3-methyl-6-methoxy-3,4-dihydro-chinazolin-4-on

a) 2-(4-Acetoxyphenyl)-6-methoxy-3,1-benzoxazin-4-on

16,7 g (0,1 Mol) 3-Methoxyanthranilsäure werden in 100 ml Pyridin gelöst und unter Kühlung und Rühren mit 23,8 g (0,1 Mol + 20%) 4-Acetoxybenzoylchlorid versetzt. Nach 2 Stunden ist die Umsetzung beendet und das Reaktionsgemisch wird auf Eiswasser gegossen. Der dabei ausgefallene Niederschlag wird abgesaugt, getrocknet und aus Methanol kristallisiert.
Schmelzpunkt: 164–166°C,
Ausbeute: 20,2 g (65 % der Theorie)

$C_{17}H_{13}NO_5$ (311,28)

| | | | |
|---|---|---|---|
| Ber.: | C 65,59 | H 4,21 | N 4,50 |
| Gef.: | C 65,51 | H 4,27 | N 4,58 |

b) 2-(4-Hydroxyphenyl)-3-methyl-6-methoxy-3,4-dihydro-chinazolin-4-on

6,2 g (0,02 Mol) des gemäß Beispiel a) erhaltenen Produktes werden mit 60 ml 40%iger Methylaminlösung in Wasser in einer Stahlbombe auf 120°C erhitzt. Nach 3 Stunden ist die Umsetzung beendet, die Reaktionslösung wird im Vakuum eingedampft und das erhaltene Rohprodukt aus Methanol kristallisiert.
Schmelzpunkt: 146–147°C,
Ausbeute: 4,8 g (85 % der Theorie)

$C_{16}H_{14}N_2O_3$ (282,30)

| | | | |
|---|---|---|---|
| Ber.: | C 68,08 | H 5,00 | N 9,92 |
| Gef.: | C 67,95 | H 5,05 | N 9,86 |

c) 2-[4-(4-Chlorbutoxy)-phenyl]-3-methyl-6-methoxy-3,4-dihydro-chinazolin-4-on

4 g (0,014 Mol) des gemäß Beispiel b) erhaltenen Produktes werden in 30 ml Dimethylsulfoxid gelöst und unter Rühren mit 1,75 g (0,014 Mol + 10%) Kalium-tert.butylat versetzt. Anschließend werden 4 ml 1-Brom-4-chlorbutan zugegeben und bei Raumtemperatur bis zur quantitativen Umsetzung gerührt. Dann wird auf Eiswasser gegossen, das kristallin ausgefallene Endprodukt abgesaugt, gut mit Wasser nachgewaschen und getrocknet.
Schmelzpunkt: 127–130°C,
Ausbeute: 4,9 g (94 % der Theorie)

$C_{20}H_{21}ClN_2O_3$ (372,85)

| | | | | |
|---|---|---|---|---|
| Ber.: | C 64,43 | H 5,68 | N 7,51 | Cl 9,51 |
| Gef.: | C 64,28 | H 5,61 | N 7,53 | Cl 9,59 |

d) 2-[4-(4-Aminobutoxy)-phenyl]-3-methyl-6-methoxy-3,4-dihydro-chinazolin-4-on

4,5 g (0,012 Mol) des gemäß Beispiel c) erhaltenen Produktes werden mit 2,2 g (0,012 Mol + 10%) 2,4-Dimethoxybenzylamin versetzt und bei 120 °C zur Reaktion belassen. Nach 2 Stunden ist die Umsetzung beendet, das erhaltene Produkt wird mit 2 N Salzsäure bei Raumtemperatur gerührt, anschließend mit 2 N Natronlauge alkalisch gestellt und mit Methylenchlorid extrahiert. Die vereinten organischen Extrakte werden über Natriumsulfat getrocknet und eingedampft. Das erhaltene Rohprodukt wird über eine Kieselsäule (Korngröße: 0,2—0,5 mm; Elutionsmittel: Methylenchlorid : Methanol = 19 : 1) gereinigt, wobei nach dem Eindampfen ein farbloses Öl resultiert.
Ausbeute: 2,6 g (61 % der Theorie)

$C_{20}H_{23}N_3O_3$ (353,42)

| | | | |
|---|---|---|---|
| Ber.: | C 67,97 | H 6,56 | N 11,89 |
| Gef.: | C 67,49 | H 6,49 | N 11,73 |

Beispiel 1

2-[4-(2-Hydroxy-3-tert.butylamino-propoxy)-phenyl]-3-methyl-6-methoxy-3,4-dihydro-chinazolin-4-on

2,8 g (10 mMol) 2-(4-Hydroxyphenyl)-3-methyl-6-methoxy-3,4-dihydro-chinazolin-4-on werden in 20 ml Dimethylsulfoxid gelöst und unter Rühren mit 1,35 g (10 mMol + 20%) Kalium-tert.butylat versetzt. Anschließend werden 2,8 ml Epibromhydrin zugegeben und bei Raumtemperatur bis zur quantitativen Umsetzung gerührt. Dann wird auf Eiswasser gegossen und das kristallin angefallene Endprodukt abgesaugt, gut mit Wasser gewaschen und getrocknet.
Schmelzpunkt: 194—196 °C,
Ausbeute: 3,0 g (89 % der Theorie)

$C_{19}H_{18}N_2O_4$ (338,37)

| | | | |
|---|---|---|---|
| Ber.: | C 67,44 | H 5,36 | N 8,28 |
| Gef.: | C 67,41 | H 5,32 | N 8,27 |

b) 2-[4-(2-Hydroxy-3-tert.butylamino-propoxy)-phenyl]-3-methyl-6-methoxy-3,4-dihydro-chinazolin-4-on

1,7 g (5 mMol) des gemäß Beispiel 1 a) erhaltenen Epoxids werden mit 17 ml tert.Butylamin in einer Stahlbombe auf 120°C erhitzt. Nach 3stündiger Reaktionsdauer wird das überschüssige Amin im Vakuum abdestilliert und der resultierende ölige Rückstand aus Aceton/Äther umkristallisiert.
Schmelzpunkt: 154—156 °C,
Ausbeute: 1,75 g (83 % der Theorie)

$C_{23}H_{29}N_3O_4$ (411,51)

| | | | |
|---|---|---|---|
| Ber.: | C 67,13 | H 7,10 | N 10,21 |
| Gef.: | C 67,10 | H 7,05 | N 10,19 |

Beispiel 2

2-[4-[2-Hydroxy-3-(2-(3,4-dimethoxyphenyl)-N-methyl-äthylamino)-propoxy]-phenyl]-3-methyl-6-methoxy-3,4-dihydro-chinazolin-4-on

1,7 g (5 mMol) des unter Beispiel 1 a erhaltenen 2-[4-(1,2-Epoxy-propoxy)-phenyl]-3-methyl-6-methoxy-3,4-dihydro-chinazolin-4-on werden mit 1,7 g 2-(3,4-Dimethoxyphenyl)-N-methyl-äthylamin bei 120°C zur Reaktion gebracht. Nach quantitativer Umsetzung wird das so erhaltene Rohprodukt über eine Kieselgelsäure (Korngröße: 0,2—0,5 mm, Elutionsmittel: Methylenchlorid/Methanol = 19 : 1) gereinigt. Das nach dem Eindampfen erhaltene Produkt wird in Aceton gelöst und mit ätherischer Salzsäure das Dihydrochlorid gefällt.
Schmelzpunkt des Dihydrochlorids: 152—155°C,
Ausbeute: 1,7 g (55 % der Theorie)

$C_{30}H_{37}Cl_2N_3O_6$ (606,56)

| | | | | |
|---|---|---|---|---|
| Ber.: | C 59,40 | H 6,15 | N 6,93 | Cl 11,69 |
| Gef.: | C 59,40 | H 6,17 | N 6,93 | Cl 11,39 |

Beispiel 3

2-[4-[3-(2-Hydroxy-3-(4-methoxyphenoxy)-propylamino)-propoxy]-phenyl]-3-methyl-6-methoxy-3,4-dihydro-chinazolin-4-on

a) 2-[4-(3-Chlorpropoxy)-phenyl]-3-methyl-6-methoxy-3,4-dihydro-chinazolin-4-on

2,8 g (10 mMol) 2-(4-Hydroxyphenyl)-3-methyl-6-methoxy-3,4-dihydro-chinazolin-4-on werden in 20 ml Dimethylsulfoxid gelöst und unter Rühren mit 1,35 g (10 mMol + 20%) Kalium-tert.-butylat versetzt. Anschließend werden 2,8 ml 1-Brom-3-chlorpropan zugegeben und bei Raumtemperatur bis zur quantitativen Umsetzung gerührt. Dann wird auf Eiswasser gegossen, mit Essigsäureäthylester extrahiert und die vereinten organischen Extrakte nach Trocknen über Natriumsulfat im Vakuum eingedampft. Dabei resultiert ein farbloses Öl, das kristallin erstarrt und aus Aceton/Äther umkristallisiert wird.
Schmelzpunkt: 114—116°C,
Ausbeute: 3,1 g (86% der Theorie)

$C_{19}H_{19}ClN_2O_3$ (358,82)

| | | | | |
|---|---|---|---|---|
| Ber.: | C 63,60 | H 5,34 | N 7,81 | Cl 9,88 |
| Gef.: | C 63,47 | H 5,28 | N 7,88 | Cl 9,74 |

b) 2-[4-[3-(2-Hydroxy-3-(4-methoxyphenoxy)-propylamino)-propoxy]-phenyl]-3-methyl-6-methoxy-3,4-dihydro-chinazolin-4-on

1,8 g (5 mMol) des gemäß Beispiel 3a) erhaltenen Propylchlorids werden mit 1,8 g 2-Hydroxy-3-(4-methoxyphenoxy)-propylamin versetzt und bei 140°C zur Reaktion belassen. Nach quantitativer Umsetzung wird das so erhaltene Rohprodukt über eine Kieselgelsäule (Korngröße: 0,2—0,5 mm, Elutionsmittel: Methylenchlorid/Methanol = 19:1) gereinigt. Das nach dem Eindampfen erhaltene farblose Öl wird aus Aceton/Äther kristallisiert.
Schmelzpunkt: 134—136°C,
Ausbeute: 1,9 g (73% der Theorie)

$C_{29}H_{33}N_3O_6$ (519,60)

| | | | |
|---|---|---|---|
| Ber.: | C 67,04 | H 6,40 | N 8,09 |
| Gef.: | C 66,96 | H 6,41 | N 8,10 |

Ähnliche Ausbeuten erhält man, wenn man die Umsetzung bei 80°C in Äthanol oder bei 110°C in Toluol durchführt.

Beispiel 4

2-[4-(2-Hydroxy-3-tert.butylamino-propoxy)-phenyl]-6-methoxy-3,4-dihydro-chinazolin-4-on

a) 2-[4-(1,2-Epoxypropoxy)-phenyl]-6-methoxy-3,4-dihydro-chinazolin-4-on

4,5 g (16,7 mMol) 2-(4-Hydroxyphenyl)-6-methoxy-3,4-dihydro-chinazolin-4-on werden in 50 ml Sulfolan gelöst und unter Rühren mit 2,55 g (16,7 mMol + 10%) Kaliumkarbonat versetzt. Nachdem eine klare Lösung eingetreten ist, wird mit 4,5 ml Epibromhydrin versetzt und bei Raumtemperatur bis zur quantitativen Umsetzung gerührt. Dann wird auf Eiswasser gegossen und das kristallin ausgefallene Endprodukt abgesaugt, gut mit Wasser nachgewaschen und getrocknet.
Schmelzpunkt: 102—105°C,
Ausbeute: 4,8 g (89% der Theorie)

$C_{18}H_{16}N_2O_4$ (324,34)

| | | | |
|---|---|---|---|
| Ber.: | C 66,66 | H 4,97 | N 8,64 |
| Gef.: | C 66,38 | H 4,92 | N 8,57 |

b) 2-[4-(2-Hydroxy-3-tert.butylamino-propoxy)-phenyl]-6-methoxy-3,4-dihydro-chinazolin-4-on

2,3 g (8,5 mMol) des gemäß Beispiel 4a) erhaltenen Epoxides werden mit 23 ml tert.Butylamin in einer Stahlbombe auf 120°C erhitzt. Nach 3stündiger Reaktionsdauer ist die Umsetzung beendet, das überschüssige Amin wird abdestilliert und das resultierende Rohkristallisat aus Aceton/Äther umkristallisiert.
Schmelzpunkt: 189—191°C,
Ausbeute: 2,2 g (64,7% der Theorie).

$C_{22}H_{27}N_3O_4$ (397,46)

| | | | |
|---|---|---|---|
| Ber.: | C 66,48 | H 6,85 | N 10,57 |
| Gef.: | C 66,16 | H 6,88 | N 10,38 |

Beispiel 5

2-[4-(3-tert.Butylamino-propoxy)-phenyl]-8-methoxy-3,4-dihydro-chinazolin-4-on

a) 2-[4-(3-Chlorpropoxy)-phenyl]-8-methoxy-3,4-dihydro-chinazolin-4-on

2,7 g (10 mMol) 2-(4-Hydroxyphenyl)-6-methoxy-3,4-dihydro-chinazolin-4-on werden in 30 ml Sulfolan gelöst und mit 1,5 g (10 mMol + 10%) Kaliumkarbonat versetzt. Zu der klaren Lösung werden 2,7 ml 1-Brom-3-chlorpropan zugegeben und anschließend wird bei Raumtemperatur bis zur quantitativen Umsetzung gerührt. Dann wird auf Eiswasser gegossen, mit Essigsäureäthylester extrahiert, die vereinten organischen Extrakte über Natriumsulfat getrocknet und eingedampft. Dabei resultiert ein farbloses Öl, welches beim Abkühlen kristallisiert.
Schmelzpunkt: 50—55°C,
Ausbeute: 3,0 g (88% der Theorie)

$C_{18}H_{17}ClN_2O_3$ (344,80)

| | | | | |
|---|---|---|---|---|
| Ber.: | C 62,70 | H 4,97 | N 8,12 | Cl 10,28 |
| Gef.: | C 62,45 | H 4,84 | N 8,07 | Cl 10,09 |

b) 2-[4-(3-tert.Butylamino-propoxy)-phenyl]-8-methoxy-3,4-dihydro-chinazolin-4-on

1,5 g (4,4 mMol) des gemäß Beispiel 5a) erhaltenen Produktes werden mit 15 ml tert.Butylamin in der Stahlbombe bei 120°C zur Reaktion belassen. Nach beendeter Umsetzung wird das überschüssige Amin im Vakuum abdestilliert und der verbleibende Rückstand über eine Kieselgelsäule (Korngröße: 0,2—0,5 mm, Elutionsmittel: Methylenchlorid/Methanol = 19 : 1) gereinigt. Das nach dem Eindampfen erhaltene farblose Öl wird in Aceton gelöst und mit ätherischer Salzsäure das Dihydrochlorid gefällt.
Schmelzpunkt des Dihydrochlorids: 133—135°C,
Ausbeute: 1,7 g (85% der Theorie)

$C_{22}H_{29}Cl_2N_3O_3$ (454,60)

| | | | | |
|---|---|---|---|---|
| Ber.: | C 58,15 | H 6,43 | N 9,25 | Cl 15,61 |
| Gef.: | C 58,03 | H 6,40 | N 9,16 | Cl 15,34 |

Beispiel 6

2-[4-[2-Hydroxy-3-(2-(2-methoxyphenyl)-äthylamino)-propoxy]-phenyl]-3-methyl-6-methoxy-3,4-dihydro-chinazolin-4-on

Hergestellt analog Beispiel 2 aus 2-[4-(1,2-Epoxypropoxy)-phenyl]-3-methyl-6-methoxy-3,4-dihydro-chinazolin-4-on und 2-(2-Methoxyphenyl)-äthylamin.
Schmelzpunkt des Dihydrochlorids: 156—158°C,
Ausbeute: 41% der Theorie

$C_{28}H_{33}Cl_2N_3O_5$ (562,50)

| | | | | |
|---|---|---|---|---|
| Ber.: | C 59,79 | H 5,91 | N 7,47 | Cl 12,61 |
| Gef.: | C 59,80 | H 5,86 | N 7,45 | Cl 12,49 |

Beispiel 7

2-[4-'(2-Hydroxy-3-diäthylamino-propoxy)-phenyl]-3-methyl-6-methoxy-3,4-dihydro-chinazolin-4-on

Hergestellt analog Beispiel 1 aus 2-[4-(1,2-Epoxypropoxy)-phenyl]-3-methyl-6-methoxy-3,4-dihydro-chinazolin-4-on und Diäthylamin.
Schmelzpunkt: 123—125°C (Aceton/Äther),
Ausbeute: 52% der Theorie.

$C_{23}H_{29}N_3O_4$ (411,51)

| | | | |
|---|---|---|---|
| Ber.: | C 67,14 | H 7,10 | N 10,21 |
| Gef.: | C 67,09 | H 7,06 | N 10,18 |

Beispiel 8

2-[4-(2-Hydroxy-3-isopropylamino-propoxy)-phenyl]-3-methyl-6-methoxy-3,4-dihydro-chinazolin-4-on

Hergestellt analog Beispiel 1 aus 2-[4-(1,2-Epoxypropoxy)-phenyl]-3-methyl-6-methoxy-3,4-dihydro-chinazolin-4-on und Isopropylamin.
Schmelzpunkt: 130—132°C,
Ausbeute: 53% der Theorie.

$C_{22}H_{27}N_3O_4$ (397,48)

| | | | |
|---|---|---|---|
| Ber.: | C 66,47 | H 6,84 | N 10,57 |
| Gef.: | C 66,39 | H 6,86 | N 10,60 |

Beispiel 9

2-[4-(2-Hydroxy-3-isopropylamino-propoxy)-phenyl]-6-methoxy-3,4-dihydro-chinazolin-4-on

Hergestellt analog Beispiel 4 aus 2-[4-(1,2-Epoxypropoxy)-phenyl]-6-methoxy-3,4-dihydro-chinazolin-4-on und Isopropylamin.
Schmelzpunkt: 198—201°C,
Ausbeute: 42% der Theorie

$C_{21}H_{25}N_3O_4$ (383,45)

| | | | |
|---|---|---|---|
| Ber.: | C 65,78 | H 6,57 | N 10,96 |
| Gef.: | C 65,59 | H 6,42 | N 10,73 |

Beispiel 10

2-[4-[2-Hydroxy-3-(2-(2-methoxyphenyl)-äthylamino)-propoxy]-phenyl]-3-methyl-8-methoxy-3,4-dihydro-chinazolin-4-on

Hergestellt analog Beispiel 2 aus 2-[4-(1,2-Epoxypropoxy)-phenyl]-3-methyl-8-methoxy-3,4-dihydro-chinazolin-4-on und 2-(2-Methoxyphenyl)-äthylamin.
Schmelzpunkt des Dihydrochlorids: 120—125°C,
Ausbeute: 62% der Theorie.

$C_{28}H_{33}Cl_2N_3O_5$ (562,50)

| | | | | |
|---|---|---|---|---|
| Ber.: | C 59,79 | H 5,91 | N 7,47 | Cl 12,61 |
| Gef.: | C 59,52 | H 5,86 | N 7,41 | Cl 12,48 |

## Beispiel 11

2-[4-(2-Hydroxy-3-diäthylamino-propoxy)-phenyl]-3-methyl-8-methoxy-3,4-dihydro-chinazolin-4-on

Hergestellt analog Beispiel 1 aus 2-[4-(1,2-Epoxypropoxy)-phenyl]-3-methyl-8-methoxy-3,4-dihydro-chinazolin-4-on und Diäthylamin.
Schmelzpunkt des Dihydrochlorids: 127—133°C,
Ausbeute: 71 % der Theorie

$C_{23}H_{31}Cl_2N_3O_4$ (484,43)

| | | | | |
|---|---|---|---|---|
| Ber.: | C 57,03 | H 6,45 | N 8,67 | Cl 14,64 |
| Gef.: | C 57,11 | H 6,41 | N 8,63 | Cl 14,28 |

## Beispiel 12

2-[4-[2-Hydroxy-3-(2-(2-methylphenoxy)-äthylamino)-propoxy]-phenyl]-3-methyl-8-methoxy-3,4-dihydro-chinazolin-4-on

Hergestellt analog Beispiel 2 aus 2-[4-(1,2-Epoxypropoxy)-phenyl]-3-methyl-8-methoxy-3,4-dihydro-chinazolin-4-on und 2-(2-Methylphenoxy)-äthylamin.
Schmelzpunkt des Hydrochlorids: 120—125°C,
Ausbeute: 45 % der Theorie.

$C_{28}H_{32}ClN_3O_5$ (526,04)

| | | | | |
|---|---|---|---|---|
| Ber.: | C 63,93 | H 6,13 | N 7,99 | Cl 6,73 |
| Gef.: | C 63,81 | H 6,04 | N 8,03 | Cl 6,84 |

## Beispiel 13

2-[4-[3-(2-Hydroxy-3-(3-methylphenoxy)-propylamino)-propoxy]-phenyl]-3-methyl-8-methoxy-3,4-dihydro-chinazolin-4-on

Hergestellt analog Beispiel 3 aus 2-[4-(3-Chlorpropoxy)-phenyl]-3-methyl-8-methoxy-3,4-dihydro-chinazolin-4-on und 2-Hydroxy-3-(2-methylphenoxy)-propylamin.
Schmelzpunkt: 138—140°C,
Ausbeute: 63 % der Theorie

$C_{29}H_{33}N_3O_5$ (503,60)

| | | | |
|---|---|---|---|
| Ber.: | C 69,16 | H 6,60 | N 8,34 |
| Gef.: | C 68,98 | H 6,64 | N 8,33 |

## Beispiel 14

2-[4-[2-Hydroxy-3-(2-(2-methoxyphenyl)-äthylamino)-propoxy]-phenyl]-3-methyl-6,7-dimethoxy-3,4-dihydro-chinazolin-4-on

Hergestellt analog Beispiel 2 aus 2-[4-(1,2-Epoxypropoxy)-phenyl]-3-methyl-6,7-dimethoxy-3,4-dihydro-chinazolin-4-on und 2-(2-Methoxyphenyl)-äthylamin.
Schmelzpunkt des Trihydrochlorids: 188—192°C,
Ausbeute: 29 % der Theorie.

$C_{29}H_{36}Cl_3N_3O_6$ (628,96)

| | | | | |
|---|---|---|---|---|
| Ber.: | C 55,37 | H 5,76 | N 6,68 | Cl 16,91 |
| Gef.: | C 55,51 | H 5,75 | N 6,67 | Cl 16,20 |

Beispiel 15

2-[4-[2-Hydroxy-3-(2-(2-methylphenoxy)-äthylamino)-propoxy]-phenyl]-3-methyl-6,7-dimethoxy-3,4-dihydro-chinazolin-4-on

Hergestellt analog Beispiel 2 aus 2-[4-(1,2-Epoxypropoxy)-phenyl]-3-methyl-6,7-dimethoxy-3,4-dihydro-chinazolin-4-on und 2-(2-Methylphenoxy)-äthylamin.
Schmelzpunkt: 195–198°C,
Ausbeute: 13% der Theorie.

$C_{29}H_{33}N_3O_6$ (519,57)

| | | | |
|---|---|---|---|
| Ber.: | C 67,03 | H 6,40 | N 8,09 |
| Gef.: | C 67,23 | H 6,53 | N 8,13 |

Beispiel 16

2-[4-(3-tert.Butylamino-propoxy)-phenyl]-3-methyl-6,7-dimethoxy-3,4-dihydro-chinazolin-4-on

Hergestellt analog Beispiel 3 aus 2-[4-(3-Chlorpropoxy)-phenyl]-3-methyl-6,7-dimethoxy-3,4-dihydro-chinazolin-4-on und tert.Butylamin.
Schmelzpunkt des Hydrochlorids: 283–286°C,
Ausbeute: 78% der Theorie.

$C_{24}H_{32}ClN_3O_4$ (461,97)

| | | | | |
|---|---|---|---|---|
| Ber.: | C 62,39 | H 6,98 | N 9,10 | Cl 7,67 |
| Gef.: | C 62,18 | H 6,92 | N 9,15 | Cl 7,80 |

Beispiel 17

2-[4-[3-(2-Hydroxy-3-(3-methylphenoxy)-propylamino)-propoxy]-phenyl]-3-methyl-6,7-dimethoxy-3,4-dihydro-chinazolin-4-on

Hergestellt analog Beispiel 3 aus 2-[4-(3-Chlorpropoxy)-phenyl]-3-methyl-6,7-dimethoxy-3,4-dihydro-chinazolin-4-on und 2-Hydroxy-3-(2-methylphenoxy)-propylamin.
Schmelzpunkt des Hydrochlorids: 222°C,
Ausbeute: 22% der Theorie.

$C_{30}H_{36}ClN_3O_6$ (570,07)

| | | | | |
|---|---|---|---|---|
| Ber.: | C 63,20 | H 6,36 | N 7,37 | Cl 6,22 |
| Gef.: | C 62,91 | H 6,51 | N 7,27 | Cl 6,50 |

Beispiel 18

2-[3-Methoxy-4-(2-hydroxy-3-tert.butylaminopropoxy)-phenyl]-3-methyl-6-methoxy-3,4-dihydro-chinazolin-4-on

Hergestellt analog Beispiel 1 aus 2-[3-Methoxy-4-(1,2-epoxypropoxy)-phenyl]-3-methyl-6-methoxy-3,4-dihydro-chinazolin-4-on und tert.Butylamin.
Schmelzpunkt: 100–105°C (Aceton/Äther)
Ausbeute: 73% der Theorie

$C_{24}H_{31}N_3O_5$ (441,53)

| | | | |
|---|---|---|---|
| Ber.: | C 65,29 | H 7,08 | N 9,52 |
| Gef.: | C 65,27 | H 7,12 | N 9,38 |

## Beispiel 19

2-[4-(2-Hydroxy-3-tert.butylaminopropoxy)-phenyl]-3-methyl-6-nitro-3,4-dihydro-chinazolin-4-on

Hergestellt analog Beispiel 1 aus 2-[4-(1,2-Epoxypropoxy)-phenyl]-3-methyl-6-nitro-3,4-dihydro-chinazolin-4-on und tert.-Butylamin.
Schmelzpunkt: 267—270°C (Aceton/Äther),
Ausbeute: 31 % der Theorie

$C_{22}H_{26}N_4O_5$ .(426,48)

| | | | |
|---|---|---|---|
| Ber.: | C 61,96 | H 6,15 | N 13,14 |
| Gef.: | C 61,83 | H 6,07 | N 12,97 |

## Beispiel 20

2-[4-(2-Hydroxy-3-tert.butylaminopropoxy)-phenyl]-3,6-dimethyl-3,4-dihydro-chinazolin-4-on

Hergestellt analog Beispiel 1 aus 2-[4-(1,2-Epoxypropoxy)-phenyl]-3,6-dimethyl-3,4-dihydro-chinazolin-4-on und tert.Butylamin.
Schmelzpunkt: 146—150°C (Aceton/Äther),
Ausbeute: 88 % der Theorie

$C_{23}H_{29}N_3O_3$ (395,51)

| | | | |
|---|---|---|---|
| Ber.: | C 69,85 | H 7,39 | N 10,62 |
| Gef.: | C 69,58 | H 7,29 | N 10,52 |

## Beispiel 21

2-[4-(2-Hydroxy-3-isopropylaminopropoxy)-phenyl]-3,6-dimethyl-3,4-dihydro-chinazolin-4-on

Hergestellt analog Beispiel 1 aus 2-[4-(1,2-Epoxypropoxy)-phenyl]-3,6-dimethyl-3,4-dihydro-chinazolin-4-on und Isopropylamin.
Schmelzpunkt: 147—153°C (Aceton/Äther),
Ausbeute: 87 % der Theorie

$C_{22}H_{27}N_3O_3$ (381,48)

| | | | |
|---|---|---|---|
| Ber.: | C 69,27 | H 7,14 | N 11,02 |
| Gef.: | C 69,21 | H 7,23 | N 11,07 |

## Beispiel 22

2-[4-(2-Hydroxy-3-tert.butylaminopropoxy)-phenyl]-3-methyl-6-chlor-3,4-dihydro-chinazolin-4-on

Hergestellt analog Beispiel 1 aus 2-[4-(1,2-Epoxypropoxy)-phenyl]-3-methyl-6-chlor-3,4-dihydro-chinazolin-4-on und tert.-Butylamin.
Schmelzpunkt: 166—168°C (Aceton/Äther),
Ausbeute: 80 % der Theorie

$C_{22}H_{26}ClN_3O_3$ (415,92)

| | | | | |
|---|---|---|---|---|
| Ber.: | C 63,53 | H 6,30 | N 10,10 | Cl 8,52 |
| Gef.: | C 64,18 | H 5,79 | N 10,67 | Cl 8,40 |

### Beispiel 23

2-[4-(3-Diäthylamino-propoxy)-phenyl]-3-methyl-3,4-dihydro-chinazolin-4-on-hydrojodid

0,9 g (0,0025 Mol) 2-[4-(3-Diäthylamino-propoxy)-phenyl]-3,4-dihydro-chinazolin-4-on löst man unter Rühren bei 60°C in 10 ml Dimethylsulfoxid, kühlt auf Raumtemperatur ab und gibt 0,34 g (0,003 Mol) Kalium-tert.butylat dazu. Nach 15 Minuten tropft man 0,9 ml Methyljodid dazu, rührte weitere 2 Stunden bei Raumtemperatur und gießt das Reaktionsgemisch auf 100 ml Aceton. Durch Rühren und Zugaben von ca. 20 ml Äther scheidet sich das gewünschte Produkt in weißen Kristallen ab, welches abgesaugt, mit Äther gewaschen und im Umlufttrockenschrank bei 50°C getrocknet wird.
Schmelzpunkt: 218–222°C,
Ausbeute: 0,8 g (67% der Theorie)

$C_{22}H_{27}N_3O_2 \times HJ$ (493,38)

| | | | | |
|---|---|---|---|---|
| Ber.: | C 53,55 | H 5,71 | N 8,51 | H 25,72 |
| Gef.: | C 53,58 | H 6,00 | N 8,21 | H 25,50 |

### Beispiel 24

2-[4-(2-Hydroxy-3-isopropylamino-propoxy)-phenyl]-3-methyl-3,4-dihydro-pyrido[2,3-e]pyrimidin-4-on-dihydrochlorid

a) 2-[4-(1,2-Epoxy-propoxy)-phenyl]-3-methyl-3,4-dihydro-pyrido[2,3-e]pyrimidin-4-on

9,4 g (0,037 Mol) 2-(4-Hydroxy-phenyl)-3-methyl-3,4-dihydro-pyrido[2,3-e]pyrimidin-4-on werden in 100 ml Dimethylsulfoxid gelöst und unter Rühren mit 4,5 g (0,04 Mol) Kalium-tert.-butylat versetzt. Nachdem eine klare Lösung eingetreten ist, wird mit 9,4 ml Epibromhydrin versetzt und eine Stunde bei Raumtemperatur nachgerührt. Dann wird auf 600 ml Eiswasser gegossen und das kristallin ausgefallene Produkt abgesaugt und im Vakuumtrockenschrank bei 40°C getrocknet.
Schmelzpunkt: 92–95°C,
Ausbeute: 9,3 g (81% der Theorie)

$C_{17}H_{15}N_3O_3$ (309,31)

| | | | |
|---|---|---|---|
| Ber.: | C 66,00 | H 4,89 | N 13,58 |
| Gef.: | C 66,13 | H 4,96 | N 13,42 |

b) 2-[4-(2-Hydroxy-3-isopropylamino-propoxy)-phenyl]-3-methyl-3,4-dihydro-pyrido[2,3-e]pyrimidin-4-on-dihydrochlorid

2,9 g (0,009 Mol) des gemäß Beispiel 24a erhaltenen Produktes werden mit 30 ml Isopropylamin in der Stahlbombe bei 120°C umgesetzt. Nach beendeter Reaktion wird das überschüssige Amin im Vakuum abdestilliert und der verbleibende Rückstand über eine Kieselgelsäule (Korngröße: 0,2–0,5 mm; Elutionsmittel: Methylenchlorid/Methanol = 19 : 1) gereinigt. Das nach dem Eindampfen erhaltene farblose Öl wird in 50 ml Aceton gelöst und mit ätherischer Salzsäure das Dihydrochlorid gefällt.
Schmelzpunkt des Dihydrochlorids: 142–145°C,
Ausbeute: 2,0 g (50% der Theorie)

$C_{20}H_{26}Cl_2N_4O_3$ (441,37)

| | | | | |
|---|---|---|---|---|
| Ber.: | C 54,42 | H 5,94 | N 12,69 | Cl 16,07 |
| Gef.: | C 54,20 | H 6,11 | N 12,87 | Cl 16,00 |

### Beispiel 25

2-[4-(2-Hydroxy-3-tert.butylamino-propoxy)-phenyl]-3-methyl-3,4-dihydro-pyrido[2,3-e]pyrimidin-4-on-dihydrochlorid

Hergestellt analog Beispiel 24 aus 2-[4-(1,2-Epoxy-propoxy)-phenyl]-3-methyl-3,4-dihydro-pyrido[2,3-e]pyrimidin-4-on und tert.Butylamin.

Schmelzpunkt des Dihydrochlorids: 168—171°C,
Ausbeute: 34% der Theorie

$C_{21}H_{28}Cl_2N_4O_3$ (455,37)

| | | | | |
|---|---|---|---|---|
| Ber.: | C 55,38 | H 6,19 | N 12,30 | Cl 15,57 |
| Gef.: | C 55,30 | H 6,25 | N 12,26 | Cl 15,52 |

## Beispiel 26

### 2-[4-[2-Hydroxy-3-(2-(4-methoxy-phenoxy)-äthylamino)-propoxy]-phenyl]-3-methyl-3,4-dihydro-pyrido[2,3-e]pyrimidin-4-on

Hergestellt analog Beispiel 24 aus 2-[4-(1,2-Epoxy-propoxy)-phenyl]-3-methyl-3,4-dihydro-pyrido[2,3-e]pyrimidin-4-on und 2-(4-Methoxy-phenoxy)-äthylamin.
Schmelzpunkt: 132—135°C,
Ausbeute: 17% der Theorie

$C_{26}H_{28}N_4O_5$ (476,51)

| | | | |
|---|---|---|---|
| Ber.: | C 65,53 | H 5,92 | N 11,75 |
| Gef.: | C 65,42 | H 6,06 | N 11,87 |

## Beispiel 27

### 2-[4-(2-Hydroxy-3-isopropylamino-propoxy)-phenyl]-3-methyl-3,4-dihydro-pyrido[3,4-e]pyrimidin-4-on-dihydrochlorid

Hergestellt analog Beispiel 24 aus 2-[4-(1,2-Epoxy-propoxy)-phenyl]-3-methyl-3,4-dihydro-pyrido[3,4-e]pyrimidin-4-on und Isopropylamin.
Schmelzpunkt des Dihydrochlorids: 122—125°C,
Ausbeute: 68% der Theorie

$C_{20}H_{26}Cl_2N_4O_3$ (441,35)

| | | | | |
|---|---|---|---|---|
| Ber.: | C 54,42 | H 5,94 | N 12,69 | Cl 16,06 |
| Gef.: | C 54,39 | H 5,88 | N 12,74 | Cl 16,02 |

## Beispiel 28

### 2-[4-(2-Hydroxy-3-tert.butylamino-propoxy)-phenyl]-3-methyl-3,4-dihydro-pyrido[3,4-e]pyrimidin-4-on-dihydrochlorid

Hergestellt analog Beispiel 24 aus 2-[4-(1,2-Epoxy-propoxy)-phenyl]-3-methyl-3,4-dihydro-pyrido[3,4-e]pyrimidin-4-on und tert.Butylamin.
Schmelzpunkt des Dihydrochlorids: 171—173°C,
Ausbeute: 66,6% der Theorie.

$C_{21}H_{28}Cl_2N_4O_3$ (455,37)

| | | | | |
|---|---|---|---|---|
| Ber.: | C 55,38 | H 6,19 | N 12,30 | Cl 15,57 |
| Gef.: | C 55,24 | H 6,24 | N 12,43 | Cl 15,70 |

## Beispiel 29

### 2-[4-[4-(2-Hydroxy-3-(4-methoxyphenoxy)-propylamino)-butoxy]-phenyl]-3-methyl-6-methoxy-3,4-dihydro-chinazolin-4-on

2,5 g (0,007 Mol) 2-[4-(4-Aminobutoxy)-phenyl]-3-methyl-6-methoxy-3,4-dihydro-chinazolin-2-on werden mit 1,5 g (0,007 Mol + 20%) 1,2-Epoxy-3-(4-methoxy-phenoxy)-propan bei 120°C umgesetzt. Nach 3 Stunden ist die Umsetzung beendet und das so erhaltene Rohprodukt wird über eine

Kieselgelsäule (Korngröße: 0,2—0,5 mm; Elutionsmittel: Methylenchlorid/Methanol = 49 : 1) gereinigt. Das nach dem Eindampfen erhaltene gelbliche Öl wird in Aceton gelöst und mit ätherischer Salzsäure das Hydrochlorid gefällt.
Schmelzpunkt des Hydrochlorids: 105—107°C,
Ausbeute: 2,3 g (57% der Theorie)

$C_{30}H_{36}ClN_3O_6$ (570,08)

| | | | | |
|---|---|---|---|---|
| Ber.: | C 63,20 | H 6,36 | N 7,37 | Cl 6,22 |
| Gef.: | C 63,12 | H 6,18 | N 7,28 | Cl 6,14 |

Beispiel 30

2-[4-[3-(2-Hydroxy-3-(4-methoxyphenoxy)-propylamino)-propoxy]-phenyl]-8-methoxy-3,4-dihydro-chinazolin-4-on

a) 2-[4-(3-Chlorpropoxy)-phenyl]-8-methoxy-3,4-dihydro-chinazolin-4-on

2,7 g (10 mMol) 2-(4-Hydroxyphenyl)-8-methoxy-3,4-dihydro-chinazolin-4-on werden in 30 ml Sulfolan gelöst und mit 1,5 g (10 mMol + 10%) Kaliumkarbonat versetzt. Zu der klaren Lösung werden 2,7 ml 1-Brom-3-chlorpropan zugegeben und anschließend wird bei Raumtemperatur bis zur quantitativen Umsetzung gerührt. Dann wird auf Eiswasser gegossen, mit Essigsäureäthylester extrahiert, die vereinten organischen Extrakte über Natriumsulfat getrocknet und eingedampft. Dabei resultiert ein farbloses Öl, welches beim Abkühlen kristallisiert.
Schmelzpunkt: 50—55°C,
Ausbeute: 3,0 g (88% der Theorie)

$C_{18}H_{17}ClN_2O_3$ (344,80)

| | | | | |
|---|---|---|---|---|
| Ber.: | C 62,70 | H 4,97 | N 8,12 | Cl 10,28 |
| Gef.: | C 62,45 | H 4,84 | N 8,07 | Cl 10,09 |

b) 2-[4-[3-(2-Hydroxy-3-(4-methoxyphenoxy)-propylamino)-propoxy]-phenyl]-8-methoxy-3,4-dihydro-chinazolin-4-on

1,5 g (4,4 mMol) der gemäß Beispiel 30a erhaltenen Substanz werden mit 1,05 g (4,4 mMol + 10%) 2-Hydroxy-3-(4-methoxy-phenoxy)-propylamin bei 120°C umgesetzt. Nach quantitativer Umsetzung wird das so erhaltene Rohprodukt über eine Kieselgelsäule (Korngröße: 0,2—0,5 mm; Elutionsmittel: Methylenchlorid/Methanol = 19 : 1) gereinigt. Das nach dem Eindampfen erhaltene gelbe Öl wird in Aceton gelöst und mit ätherischer Salzsäure das Hydrochlorid gefällt.
Schmelzpunkt des Dihydrochlorids: 190—193°C,
Ausbeute: 1,4 g (56% der Theorie)

$C_{28}H_{33}Cl_2N_3O_6$ (578,60)

| | | | | |
|---|---|---|---|---|
| Ber.: | C 58,12 | H 5,75 | N 7,26 | Cl 12,26 |
| Gef.: | C 58,34 | H 5,71 | N 7,34 | Cl 11,98 |

Beispiel 31

2-[4-(2-Hydroxy-3-tert.butylamino-propoxy)-phenyl]-3-isopropyl-6-methoxy-3,4-dihydro-chinazolin-4-on

Hergestellt analog Beispiel 1 aus 2-[4-(1,2-Epoxypropoxy)-phenyl]-3-isopropyl-6-methoxy-3,4-dihydro-chinazolin-4-on und tert.Butylamin.
Schmelzpunkt: 145—147°C,
Ausbeute: 78% der Theorie

$C_{25}H_{33}N_3O_4$ (439,56)

| | | | |
|---|---|---|---|
| Ber.: | C 65,62 | H 7,41 | N 9,18 |
| Gef.: | C 65,49 | H 7,88 | N 9,20 |

Beispiel 32

2-[4-[2-Hydroxy-3-(2-(3,4-dimethoxyphenyl)-N-propyl-äthylamino)-propoxy]-phenyl]-3-methyl-6-methoxy-3,4-dihydro-chinazolin-4-on

Hergestellt analog Beispiel 2 aus 2-[4-(1,2-Epoxypropoxy)-phenyl]-3-methyl-6-methoxy-3,4-dihydro-chinazolin-4-on und 2-(3,4-Dimethoxyphenyl)-N-propyl-äthylamin.
Schmelzbereich: 112–117°C,
Ausbeute: 40% der Theorie

$C_{32}H_{41}Cl_2N_3O_6$ (634,61)

| | | | | |
|---|---|---|---|---|
| Ber.: | C 60,56 | H 6,51 | N 6,62 | Cl 11,17 |
| Gef.: | C 59,89 | H 6,79 | N 6,70 | Cl 11,15 |

Im folgenden wird die Erfindung anhand galenischer Beispiele erläutert:

Beispiel 33

Tabletten zu 100 mg 2-[4-(2-Hydroxy-3-tert.butylamino-propoxy)-phenyl]-3-methyl-6-methoxy-3,4-dihydro-chinazolin-4-on

Zusammensetzung:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Herstellungsverfahren

Der Wirkstoff und der Milchzucker werden gleichmäßig mit einer wäßrigen Lösung des Polyvinylpyrrolidons befeuchtet und granuliert. Nach dem Trocknen wird das Granulat mit den restlichen Wirkstoffen vermischt und die Mischung in üblicher Weise zu Tabletten verpreßt.

Beispiel 34

Suppositorien zu 150 mg (2-[4-(2-Hydroxy-3-tert.butylamino-propoxy)-phenyl]-3-methyl-6-methoxy-3,4-dihydro-chinazolin-4-on

Zusammensetzung:

| | |
|---|---|
| Wirkstoff | 150,0 mg |
| Suppositorienmasse | 1550,0 mg |
| | 1700,0 mg |

Herstellungsverfahren

Der Wirkstoff wird in die geschmolzene Suppositorienmasse gleichmäßig eingerührt und suspendiert und das flüssige Gemisch in gekühlte Suppositorienformen ausgegossen.

Beispiel 35

Dragées zu 50 mg 2-[4-(2-Hydroxy-3-tert.butylamino-propoxy)-phenyl]-3-methyl-6-methoxy-3,4-dihydro-chinazolin-4-on

1 Dragéekern enthält:

| | |
|---|---|
| Wirksubstanz | 50,0 mg |
| Maisstärke getrocknet | 20,0 mg |
| lösliche Stärke | 2,0 mg |
| Carboxymethylcellulose | 7,0 mg |
| Magnesiumstearat | 1,0 mg |
| | 80,0 mg |

Herstellungsverfahren

Das Gemisch wird wie in Beispiel A beschrieben zu Dragéekernen verarbeitet, die dann mit Zucker und Gummi-Arabikum dragiert werden.

Beispiel 36

Suspension mit 250 mg 2-[4-(2-Hydroxy-3-tert.butylamino-propoxy)-phenyl]-3-methyl-6-methoxy-3,4-dihydro-chinazolin-4-on

100 ml Suspension enthalten:

| | |
|---|---|
| Wirksubstanz | 5,0 g |
| Carboxymethylcellulose | 0,1 g |
| p-Hydroxybenzoesäuremethylester | 0,05 g |
| p-Hydroxybenzoesäurepropylester | 0,01 g |
| Zucker | 10,0 g |
| Glycerin | 5,0 g |
| Sorbitlösung 70% | 20,0 g |
| Aroma | 0,3 g |
| destilliertes Wasser | ad 100,0 ml |

Herstellungsverfahren

In dem auf 70°C erhitzten destillierten Wasser wird unter Rühren p-Hydroxybenzoesäuremethylester und -propylester sowie Glycerin und Carboxymethylcellulose gelöst. Die Lösung wird auf Raumtemperatur abgekühlt und unter Rühren der Wirkstoff zugegeben und homogen dispergiert. Nach Zugabe und Lösen des Zucker, der Sorbitlösung und des Aromas wird die Suspension zur Entlüftung unter Rühren evakuiert.

**Patentansprüche für die Vertragsstaaten: BE, CH, DE, FR, IT, LI, LU, NL, SE**

1. Neue Pyrimidinone der allgemeinen Formel

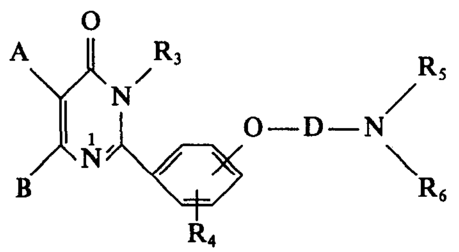

(I)

in der

A und B zusammen mit den beiden dazwischenliegenden Kohlenstoffatomen einen Pyridinring oder einen Phenylring, der durch die Reste $R_1$ und/oder $R_2$ substituiert sein kann, wobei

$R_1$ ein Halogenatom, eine Amino- oder Nitrogruppe, eine Alkyl- oder Alkoxygruppe mit jeweils 1 bis 3 Kohlenstoffatomen und
$R_2$ eine Alkoxygruppe mit 1 bis 3 Kohlenstoffatomen darstellen,

D eine gegebenenfalls durch eine Hydroxygruppe substituierte Alkylengruppe mit 3 bis 4 Kohlenstoffatomen,
$R_3$ und $R_5$, die gleich oder verschieden sein können, je ein Wasserstoffatom oder eine Alkylgruppe mit 1 bis 3 Kohlenstoffatomen,
$R_4$ ein Wasserstoffatom oder eine Alkoxygruppe mit 1 bis 3 Kohlenstoffatomen und
$R_6$ eine geradkettige oder verzweigte Alkylgruppe mit 1 bis 6 Kohlenstoffatomen oder eine gegebenenfalls durch eine Hydroxygruppe substituierte geradkettige gesättigte Alkylengruppe mit 2 bis 4 Kohlenstoffatomen, welche endständig durch eine Phenyl- oder Phenoxygruppe substituiert ist, wobei der Phenylkern jeweils durch Alkyl- und/oder Alkoxygruppen mit jeweils 1 bis 3 Kohlenstoffatomen mono- oder disubstituiert sein kann, bedeuten, und deren Säureadditionssalze.

2. Neue Pyrimidinone der allgemeinen Formel I gemäß Anspruch 1, in der

A und B zusammen mit den beiden dazwischenliegenden Kohlenstoffatomen, einen Pyridinring oder einen Phenylring, der durch die Reste $R_1$ und/oder $R_2$ substituiert sein kann, wobei $R_1$ ein Chloratom, eine Methyl-, Methoxy- oder Nitrogruppe und
$R_2$ eine Methoxygruppe darstellen,
D eine n-Propylen-, n-Butylen-, 2-Hydroxy-n-propylen-, 2-Hydroxy-n-butylen- oder 3-Hydroxy-n-butylengruppe,
$R_3$ und $R_5$, die gleich oder verschieden sein können, je ein Wasserstoffatom oder eine Alkylgruppe mit 1 bis 3 Kohlenstoffatomen,
$R_4$ ein Wasserstoffatom oder die Methoxygruppe und
$R_6$ eine Alkylgruppe mit 1 bis 4 Kohlenstoffatomen, eine in 2-Stellung durch eine Methoxyphenyl-, Dimethoxyphenyl-, Methylphenoxy- oder Methoxyphenoxygruppe substituierte Äthylgruppe oder eine in 3-Stellung durch eine Methoxyphenoxy- oder Methylphenoxygruppe substituierte 2-Hydroxypropylgruppe bedeuten, und deren Säureadditionssalze, insbesondere deren physiologisch verträgliche Säureadditionssalze mit anorganischen oder organischen Säuren.

3. Neue Pyrimidinone der allgemeinen Formel I gemäß Anspruch 1, in der

A und B zusammen mit den beiden dazwischenliegenden Kohlenstoffatomen einen Phenyl-, Methoxyphenyl-, Dimethoxyphenyl- oder Pyridinring,
D die n-Propylen- oder 2-Hydroxy-n-propylengruppe,
$R_3$ ein Wasserstoffatom oder die Methylgruppe,
$R_4$ ein Wasserstoffatom oder die Methoxygruppe,
$R_5$ ein Wasserstoffatom oder eine Alkylgruppe mit 1 bis 3 Kohlenstoffatomen und
$R_6$ eine Alkylgruppe mit 1 bis 4 Kohlenstoffatomen, eine in 2-Stellung durch eine Methoxyphenyl-, Dimethoxyphenyl-, Methylphenoxy- oder Methoxyphenoxygruppe substituierte Äthylgruppe oder eine in 3-Stellung durch eine Methoxyphenoxy- oder Methylphenoxygruppe substituierte 2-Hydroxypropylgruppe bedeuten, und deren physiologisch verträgliche Säureadditionssalze mit anorganischen oder organischen Säuren.

4. Neue Pyrimidinone der allgemeinen Formel

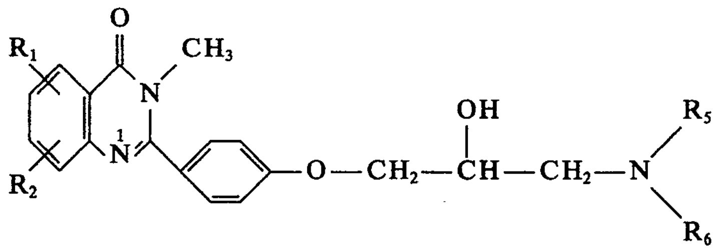

(Ia)

in der

$R_1$ in 6- oder 8-Stellung eine Methoxygruppe,
$R_2$ ein Wasserstoffatom oder in 7-Stellung eine Methoxygruppe und
$R_5$ und $R_6$ zusammen mit dem dazwischenliegenden Stickstoffatom eine Isopropylamino-, tert.Butylamino-, N-Methyl-2-(3,4-dimethoxyphenyl)-äthylamino-, 2-(2-Methoxyphenyl)-äthylamino-, 2-(2-Methylphenoxy)-äthylamino- oder 2-(4-Methoxyphenoxy)-äthylaminogruppe bedeuten, und deren physiologisch verträgliche Säureadditionssalze mit anorganischen oder organischen Säuren.

5. 2-[4-(2-Hydroxy-3-tert.butylamino-propoxy)-phenyl]-3-methyl-6-methoxy-3,4-dihydro-chinazolin-4-on und dessen Säureadditionssalze.

6. 2-[4-[2-Hydroxy-3-(2-(3,4-dimethoxyphenyl)-N-methyl-äthylamino)-propoxy]-phenyl]-3-methyl-6-methoxy-3,4-dihydrochinazolin-4-on und dessen Säureadditionssalze.

7. 2-[4-[2-Hydroxy-3-(2-(2-methoxyphenyl)-äthylamino)-propoxy]-phenyl]-3-methyl-6-methoxy-3,4-dihydro-chinazolin-4-on und dessen Säureadditionssalze.

8. Arzneimittel, enthaltend eine Verbindung gemäß den Ansprüchen 1 bis 7 neben einem oder mehreren inerten Trägerstoffen und/oder Verdünnungsmitteln.

9. Verwendung einer Verbindung gemäß den Ansprüchen 1 bis 7 zur Herstellung eines Arzneimittels auf nichtchemischem Wege zur Bekämpfung des Hochdrucks und von Coronarerkrankungen.

10. Verfahren zur Herstellung von neuen Pyrimidinonen der allgemeinen Formel

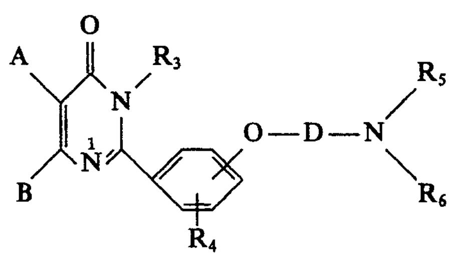

(I)

in der

A und B zusammen mit den beiden dazwischenliegenden Kohlenstoffatomen einen Pyridinring oder einen Phenylring, der durch die Reste $R_1$ und/oder $R_2$ substituiert sein kann, wobei

$R_1$ ein Halogenatom, eine Amino- oder Nitrogruppe, eine Alkyl- oder Alkoxygruppe mit jeweils 1 bis 3 Kohlenstoffatomen und
$R_2$ eine Alkoxygruppe mit 1 bis 3 Kohlenstoffatomen darstellen,

D eine gegebenenfalls durch eine Hydroxygruppe substituierte Alkylengruppe mit 3 oder 4 Kohlenstoffatomen,
$R_3$ und $R_5$, die gleich oder verschieden sein können, je ein Wasserstoffatom oder eine Alkylgruppe mit 1 bis 3 Kohlenstoffatomen,
$R_4$ ein Wasserstoffatom oder eine Alkoxygruppe mit 1 bis 3 Kohlenstoffatomen und
$R_6$ eine geradkettige oder verzweigte Alkylgruppe mit 1 bis 6 Kohlenstoffatomen oder eine gegebenenfalls durch eine Hydroxygruppe substituierte geradkettige gesättigte Alkylengruppe mit 2 bis 4 Kohlenstoffatomen, welche endständig durch eine Phenyl- oder Phenoxygruppe substituiert ist, wobei der Phenylkern jeweils durch Alkyl- und/oder Alkoxygruppen mit jeweils 1 bis 3 Kohlenstoffatomen mono- oder disubstituiert sein kann, bedeuten und von deren Säureadditionssalzen, dadurch gekennzeichnet, daß

a) ein Propoxyphenylderivat der allgemeinen Formel

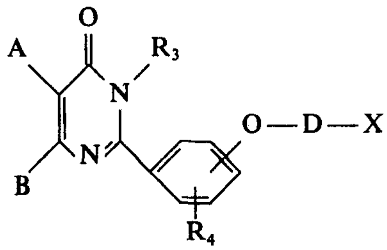

(II)

in der
A, B, D, $R_3$ und $R_4$ wie eingangs definiert sind, X eine nukleophil austauschbare Gruppe oder X zusammen mit einem $\beta$-ständigen Wasserstoffatom des Restes D ein Sauerstoffatom darstellt, mit einem Amin der allgemeinen Formel

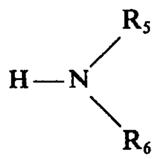

(III)

in der
$R_5$ und $R_6$ wie eingangs definiert sind, umgesetzt wird oder

b) eine Verbindung der allgemeinen Formel

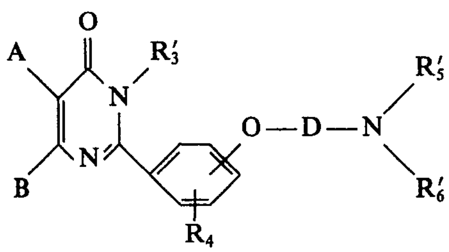

(IV)

in der
A, B, D und $R_4$ wie eingangs definiert sind, mindestens einer der Reste $R'_3$, $R'_5$ oder $R'_6$ ein Wasserstoffatom darstellt und die übrigen der Reste $R'_3$, $R'_5$ oder $R'_6$ die für $R_3$, $R_5$ oder $R_6$ eingangs erwähnten Bedeutungen besitzen, mit einer Verbindung der allgemeinen Formel

Z—Y (V)

in der
Z eine nukleophil austauschbare Gruppe oder Z zusammen mit einem $\beta$-ständigen Wasserstoffatom des Restes $R_6$ ein Sauerstoffatom darstellt und
Y mit Ausnahme der eines Wasserstoffatoms die für $R_3$, $R_5$ und $R_6$ eingangs erwähnten Bedeutungen besitzt, oder mit Formaldehyd/Ameisensäure alkyliert wird und gewünschtenfalls anschließend eine so erhaltene Verbindung der allgemeinen Formel I in ihr Säureadditionssalz, insbesondere in ihr physiologisch verträgliches Säureadditionssalz, mit anorganischen oder organischen Säuren übergeführt wird.

**Patentansprüche für den Vertragsstaat: AT**

1. Verfahren zur Herstellung von neuen Pyrimidinonen der allgemeinen Formel

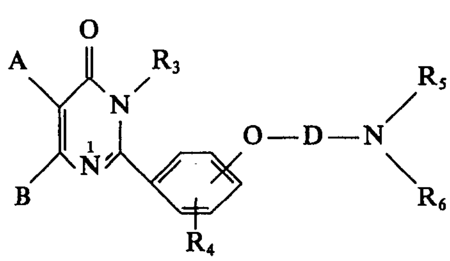

(I)

in der

A und B zusammen mit den beiden dazwischenliegenden Kohlenstoffatomen einen Pyridinring oder einen Phenylring, der durch die Reste $R_1$ und/oder $R_2$ substituiert sein kann, wobei

$R_1$ ein Halogenatom, eine Amino- oder Nitrogruppe, eine Alkyl- oder Alkoxygruppe mit jeweils 1 bis 3 Kohlenstoffatomen und
$R_2$ eine Alkoxygruppe mit 1 bis 3 Kohlenstoffatomen darstellen,

D eine gegebenenfalls durch eine Hydroxygruppe substituierte Alkylengruppe mit 3 bis 4 Kohlenstoffatomen,
$R_3$ und $R_5$, die gleich oder verschieden sein können, je ein Wasserstoffatom oder eine Alkylgruppe mit 1 bis 3 Kohlenstoffatomen,
$R_4$ ein Wasserstoffatom oder eine Alkoxygruppe mit 1 bis 3 Kohlenstoffatomen und
$R_6$ eine geradkettige oder verzweigte Alkylgruppe mit 1 bis 6 Kohlenstoffatomen oder eine gegebenenfalls durch eine Hydroxygruppe substituierte geradkettige gesättigte Alkylengruppe mit 2 bis 4 Kohlenstoffatomen, welche endständig durch eine Phenyl- oder Phenoxygruppe substituiert ist, wobei der Phenylkern jeweils durch Alkyl- und/oder Alkoxygruppen mit jeweils 1 bis 3 Kohlenstoffatomen mono- oder disubstituiert sein kann, bedeuten und von deren Säureadditionssalzen, dadurch gekennzeichnet, daß

a) ein Propoxyphenylderivat der allgemeinen Formel

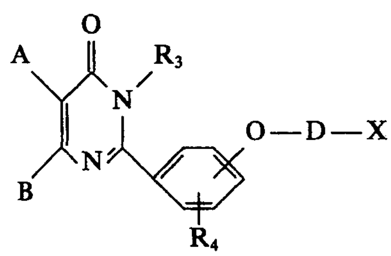

(II)

in der
A, B, D, $R_3$ und $R_4$ wie eingangs definiert sind,
X eine nukleophil austauschbare Gruppe oder X zusammen mit einem $\beta$-ständigen Wasserstoffatom des Restes D ein Sauerstoffatom darstellt, mit einem Amin der allgemeinen Formel

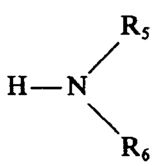

(III)

in der
$R_5$ und $R_6$ wie eingangs definiert sind, umgesetzt wird oder

b) eine Verbindung der allgemeinen Formel

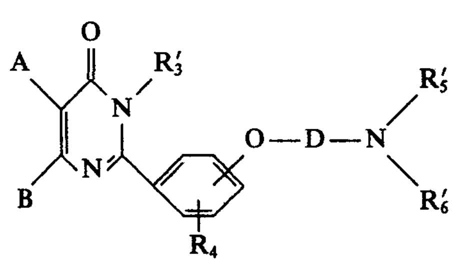

(IV)

in der
A, B, D und $R_4$ wie eingangs definiert sind, mindestens einer der Reste $R_3'$, $R_5'$ oder $R_6'$ ein Wasserstoffatom darstellt und die übrigen der Reste $R_3'$, $R_5'$ oder $R_6'$ die für $R_3$, $R_5$ oder $R_6$ eingangs erwähnten Bedeutungen besitzen, mit einer Verbindung der allgemeinen Formel

$$Z—Y \qquad (V)$$

in der
Z eine nukleophil austauschbare Gruppe oder Z zusammen mit einem β-ständigen Wasserstoffatom des Restes $R_6$ ein Sauerstoffatom darstellt und
Y mit Ausnahme der eines Wasserstoffatoms die für $R_3$, $R_5$ und $R_6$ eingangs erwähnten Bedeutungen besitzt, oder mit Formaldehyd/Ameisensäure alkyliert wird und gewünschtenfalls anschließend eine so erhaltene Verbindung der allgemeinen Formel I in ihr Säureadditionssalz, insbesondere in ihr physiologisch verträgliches Säureadditionssalz, mit anorganischen oder organischen Säuren übergeführt wird.

2. Verfahren nach Anspruch 1 zur Herstellung von neuen Pyrimidinonen der allgemeinen Formel

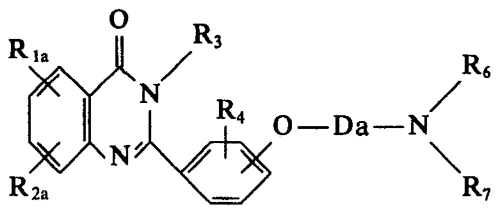

(Ia)

in der

$R_3$, $R_4$, $R_6$ und $R_7$ wie im Anspruch 1 definiert sind,
$R_{1a}$ ein Wasserstoff- oder Halogenatom, eine Amino- oder Nitrogruppe, eine Alkyl- oder Alkoxygruppe mit jeweils 1 bis 3 Kohlenstoffatomen,
$R_{2a}$ ein Wasserstoffatom oder eine Alkoxygruppe mit 1 bis 3 Kohlenstoffatomen und
Da eine gegebenenfalls in 2-Stellung durch eine Hydroxygruppe substituierte n-Propylengruppe bedeuten und von deren Säureadditionssalzen, dadurch gekennzeichnet, daß

a) ein Propoxyphenylderivat der allgemeinen Formel

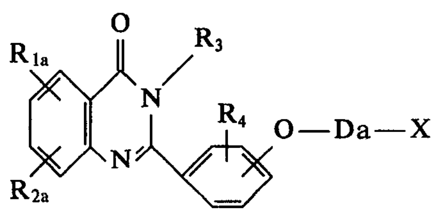

(IIa)

in der
$R_{1a}$, $R_{2a}$, $R_3$, $R_4$ und Da wie eingangs definiert sind,
X eine nukleophil austauschbare Gruppe oder X zusammen mit der 2-Hydroxygruppe ein Sauerstoffatom darstellt, mit einem Amin der allgemeinen Formel

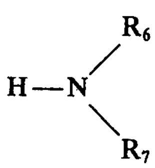

(III)

in der
$R_6$ und $R_7$ wie eingangs definiert sind, umgesetzt wird oder

b) eine Verbindung der allgemeinen Formel

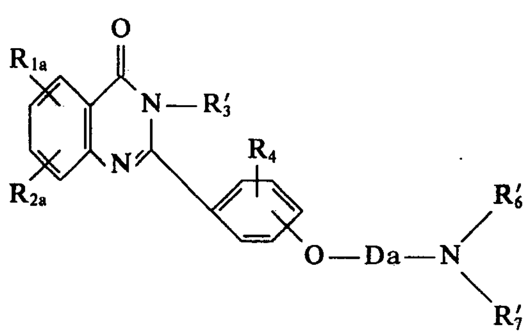

(IVa)

in der
$R_{1a}$, $R_{2a}$, $R_4$ und Da wie eingangs definiert sind und die Reste $R_3'$, $R_6'$ und $R_7'$ die im Anspruch 1 erwähnten Bedeutungen besitzen, mit einer Verbindung der allgemeinen Formel

$$Z—Y \qquad (V)$$

in der
Z und Y wie im Anspruch 1 definiert sind, oder mit Formaldehyd/Ameisensäure alkyliert wird und gewünschtenfalls anschließend eine so erhaltene Verbindung der allgemeinen Formel I in ihr Säureadditionssalz, insbesondere in ihr physiologisch verträgliches Säureadditionssalz, mit anorganischen oder organischen Säuren übergeführt wird.
Priorität: 12. 12. 1980 (DE-P 3 046 871.4)

3. Verfahren nach Anspruch 1 zur Herstellung von neuen Pyrimidinonen der allgemeinen Formel

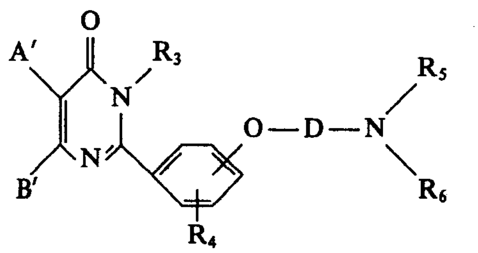

(Ib)

in der
$R_3$ bis $R_6$ und D wie im Anspruch 1 definiert sind,
A' und B' zusammen mit den beiden dazwischenliegenden Kohlenstoffatomen einen Pyridinring oder auch einen Methoxyphenylring, wenn D eine gegebenenfalls durch eine Hydroxygruppe substituierte Butylengruppe oder die Propylengruppe und die Gruppe

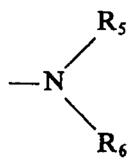

die 2-Hydroxy-3-(4-methoxyphenoxy)-propylaminogruppe darstellt, bedeuten und von deren Säureadditionssalzen, dadurch gekennzeichnet, daß

a) ein Propoxyphenylderivat der allgemeinen Formel

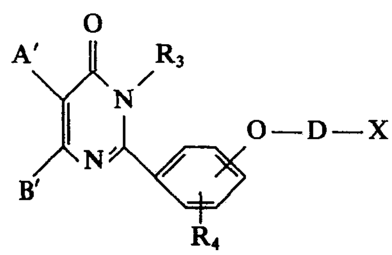

(IIb)

in der
A', B', D, $R_3$ und $R_4$ wie eingangs definiert sind und X wie im Anspruch 1 definiert ist, mit einem Amin der allgemeinen Formel

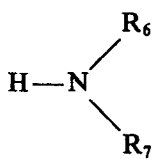

(III)

in der
$R_6$ und $R_7$ wie eingangs definiert sind, umgesetzt wird oder

b) eine Verbindung der allgemeinen Formel

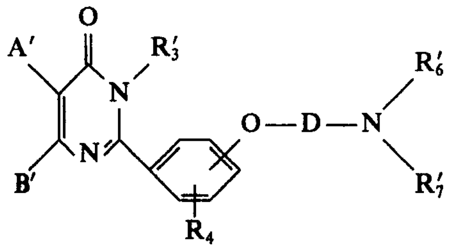

(IVb)

in der
A' und B' wie eingangs und D, $R_4$, $R'_3$, $R'_6$ und $R'_7$ wie im Anspruch 1 definiert sind, mit einer Verbindung der allgemeinen Formel

$$Z—Y \quad (V)$$

in der
Z und Y wie im Anspruch 1 definiert sind oder mit Formaldehyd/Ameisensäure alkyliert wird und gewünschtenfalls anschließend eine so erhaltene Verbindung der allgemeinen Formel I in ihr Säureadditionssalz, insbesondere in ihr physiologisch verträgliches Säureadditionssalz, mit anorganischen oder organischen Säuren übergeführt wird.
Priorität: 16.04.1981 (DE-P 3115447.6)

4. Verfahren gemäß den Ansprüchen 1 a, 2 a und 3 a, dadurch gekennzeichnet, daß die Umsetzung bei Temperaturen zwischen 50 und 200°C, vorzugsweise jedoch bei Temperaturen zwischen 70 und 150°C, durchgeführt wird.

5. Verfahren gemäß den Ansprüchen 1, 2, 3 und 4, dadurch gekennzeichnet, daß die Umsetzung in einem Lösungsmittel durchgeführt wird.

6. Verfahren gemäß den Ansprüchen 1 a, 2 a, 3 a und 4, dadurch gekennzeichnet, daß die Umsetzung in Gegenwart eines säurebindenden Mittels durchgeführt wird.

7. Verfahren gemäß den Ansprüchen 1 b, 2 b, 3 b und 5, dadurch gekennzeichnet, daß die Umsetzung bei Temperaturen zwischen 0 und 180°C, vorzugsweise jedoch bei der Siedetemperatur des Reaktionsgemisches, durchgeführt wird.

8. Verfahren gemäß den Ansprüchen 1 b, 2 b, 3 b, 5 und 7, dadurch gekennzeichnet, daß die Umsetzung in Gegenwart einer anorganischen oder tertiären organischen Base durchgeführt wird.

**Claims for the contracting states: BE, CH, DE, FR, IT, LI, LU, NL, SE**

1. New pyrimidinones of general formula

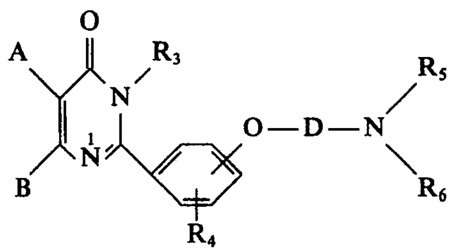

(I)

wherein

A and B together with the two carbon atoms to which they are attached represent a pyridine ring or a phenyl ring, which may be substituted by the radicals $R_1$ and/or $R_2$, wherein

- $R_1$ represents a halogen atom, an amino or nitro group, an alkyl or alkoxy group with 1 to 3 carbon atoms each, and
- $R_2$ represents an alkoxy group with 1 to 3 carbon atoms;

D represents an alkylene group with 3 or 4 carbon atoms optionally substituted by a hydroxy group;
$R_3$ and $R_5$, which may be the same or different, each represent a hydrogen atom or an alkyl group with 1 to 3 carbon atoms;
$R_4$ represents a hydrogen atom or an alkoxy group with 1 to 3 carbon atoms; and
$R_6$ represents a straight-chained or branched alkyl group with 1 to 6 carbon atoms or a straight-chained saturated alkylene group with 2 to 4 carbon atoms, optionally substituted by a hydroxy

group, which is substituted at the terminal position by a phenyl or phenoxy group, whilst the phenyl nucleus in each case may be mono- or disubstituted by alkyl and/or alkoxy groups with 1 to 3 carbon atoms each, and their acid addition salts.

2. New pyrimidinones of general formula I as claimed in claim 1, wherein

A and B together with the two carbon atoms to which they are attached represent a pyridine ring or a phenyl ring, which may be substituted by the radicals $R_1$ and/or $R_2$, whilst $R_1$ represents a chlorine atom, a methyl, methoxy, or nitro group and $R_2$ represents a methoxy group;
D represents a n-propylene, n-butylene, 2-hydroxy-n-propylene, 2-hydroxy-n-butylene or 3-hydroxy-n-butylene group;
$R_3$ and $R_5$, which may be the same or different, each represent a hydrogen atom or an alkyl group with 1 to 3 carbon atoms;
$R_4$ represents a hydrogen atom or the methoxy group; and
$R_6$ represents an alkyl group with 1 to 4 carbon atoms, an ethyl group substituted in the 2 position by a methoxyphenyl, dimethoxyphenyl, methylphenoxy or methoxyphenoxy group, or a 2-hydroxypropyl group substituted in the 3 position by a methoxyphenoxy or methylphenoxy group, and their acid addition salts, especially their physiologically compatible acid addition salts with inorganic or organic acids.

3. New pyrimidinones of general formula I as claimed in claim 1, wherein

A and B together with the two carbon atoms to which they are attached represent a phenyl, methoxyphenyl, dimethoxyphenyl or pyridine ring;
D represents the n-propylene or 2-hydroxy-n-propylene group;
$R_3$ represents a hydrogen atom or the methyl group;
$R_4$ represents a hydrogen atom or the methoxy group;
$R_5$ represents a hydrogen atom or an alkyl group with 1 to 3 carbon atoms; and
$R_6$ represents an alkyl group with 1 to 4 carbon atoms, an ethyl group substituted in the 2 position by a methoxyphenyl, dimethoxyphenyl, methylphenoxy or methoxyphenoxy group, or a 2-hydroxypropyl group substituted in the 3 position by a methoxyphenoxy or methylphenoxy group, and their physiologically compatible acid addition salts with inorganic or organic acids.

4. New pyrimidinones of general formula

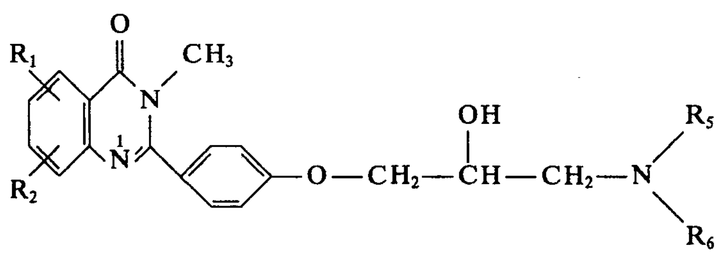

(Ia)

wherein

$R_1$ represents a methoxy group in the 6 or 8 position;
$R_2$ represents a hydrogen atom or, in the 7 position, a methoxy group; and
$R_5$ and $R_6$ together with the nitrogen atom to which they are attached represent an isopropylamino, tert.butylamino, N-methyl-2-(3,4-dimethoxyphenyl)-ethylamino, 2-(2-methoxyphenyl)-ethylamino, 2-(2-methylphenoxy)-ethylamino, or 2-(4-methoxyphenoxy)-ethylamino group, and their physiologically compatible acid addition salts with inorganic or organic acids.

5. 2-[4-(2-Hydroxy-3-tert.butylamino-propoxy)-phenyl]-3-methyl-6-methoxy-3,4-dihydroquinazolin-4-one and the acid addition salts thereof.

6. 2-[4-[2-Hydroxy-3-(2-(3,4-dimethoxyphenyl)-N-methyl-ethylamino)-propoxy]-phenyl]-3-methyl-6-methoxy-3,4-dihydroquinazolin-4-one and the acid addition salts thereof.

7. 2-[4-[2-Hydroxy-3-(2-(-methoxyphenyl)-ethylamino)-propoxy]-phenyl]-3-methyl-6-methoxy-3,4-dihydroquinazolin-4-one and the acid addition salts thereof.

8. Pharmaceutical compositions containing a compound as claimed in claims 1 to 7, besides one or more inert carriers and/or diluting agents.

9. The use of a compound as claimed in claims 1 to 7, for the preparation of a pharmaceutical composition by non-chemical manner for the treatment of hypertension and coronary diseases.

10. A process for the preparation of new pyrimidinones of general formula

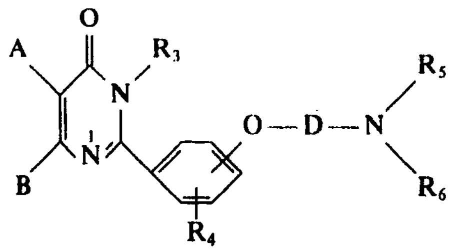

(I)

wherein

A and B together with the carbon atoms to which they are attached represent a pyridine ring or a phenyl ring, which may be substituted by the radicals $R_1$ and/or $R_2$, whilst

- $R_1$ represents a halogen atom, an amino or nitro group, an alkyl or alkoxy group with 1 to 3 carbon atoms each, and
- $R_2$ represents an alkoxy group with 1 to 3 carbon atoms;

D represents an alkylene group with 3 or 4 carbon atoms, optionally substituted by a hydroxy group;
$R_3$ and $R_5$, which may be the same or different, each represent a hydrogen atom or an alkyl group with 1 to 3 carbon atoms;
$R_4$ represents a hydrogen atom or an alkoxy group with 1 to 3 carbon atoms; and
$R_6$ represents a straight-chained or branched alkyl group with 1 to 6 carbon atoms or a straight-chained saturated alkylene group with 2 to 4 carbon atoms, optionally substituted by a hydroxy group, which is substituted at the terminal position by a phenyl or phenoxy group, whilst the phenyl nucleus is mono- or disubstituted in each case by alkyl and/or alkoxy groups with 1 to 3 carbon atoms each, and of the acid additions salts thereof, characterised in that

a) a propoxyphenyl derivative of general formula

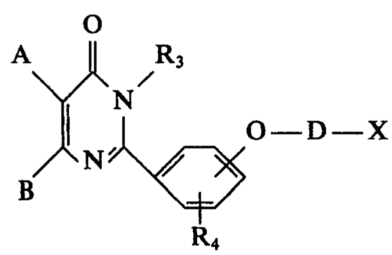

(II)

wherein
A, B, D, $R_3$ and $R_4$ are defined as mentioned before,
X represents a nucleophilically exchangeable group or
X together with the hydrogen atom in the $\beta$ position of the radical D represents an oxygen atom, is reacted with an amine of general formula

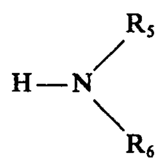

(III)

wherein
$R_5$ and $R_6$ are defined as mentioned before, or

b) a compound of general formula

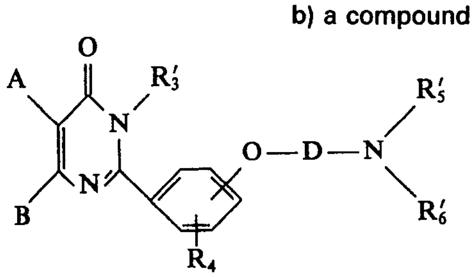

(IV)

wherein
A, B, D and $R_4$ are defined as mentioned before, at least one of the radicals $R'_3$, $R'_5$ or $R'_6$ represents a hydrogen atom and the remaining radicals $R'_3$, $R'_5$ or $R'_6$ have the meanings mentioned before for $R_3$, $R_5$ or $R_6$, is alkylated with a compound of general formula

$$Z—Y \quad (V)$$

wherein
Z represents a nucleophilically exchangeable group or Z together with a hydrogen atom in the $\beta$ position of the radical $R_6$ represents an oxygen atom, and
Y has the meanings mentioned before for $R_3$,
$R_5$ and $R_6$, with the exception of a hydrogen atom, or with formaldehyde/formic acid, and subsequently, if desired a compound of general formula I thus obtained is converted into an acid addition salt thereof, especially a physiologically compatible acid addition salt thereof, with inorganic or organic acids.

**Claims for the contracting state: AT**

1. A process for the preparation of new pyrimidinones of general formula

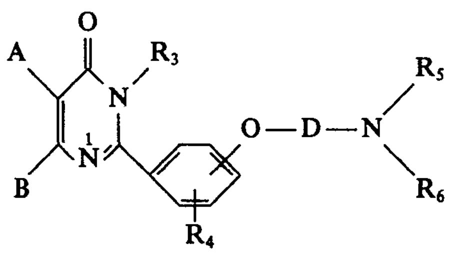

(I)

wherein

A and B together with the carbon atoms to which they are attached represent a pyridine ring or a phenyl ring, which may be substituted by the radicals $R_1$ and/or $R_2$, whilst

$R_1$ represents a halogen atom, an amino or nitro group, an alkyl or alkoxy group with 1 to 3 carbon atoms each, and
$R_2$ represents an alkoxy group with 1 to 3 carbon atoms;

D represents an alkylene group with 3 or 4 carbon atoms, optionally substituted by a hydroxy group;
$R_3$ and $R_5$, which may be the same or different, each represent a hydrogen atom or an alkyl group with 1 to 3 carbon atoms;
$R_4$ represents a hydrogen atom or an alkoxy group with 1 to 3 carbon atoms; and
$R_6$ represents a straight-chained or branched alkyl group with 1 to 6 carbon atoms or a straight-chained saturated alkylene group with 2 to 4 carbon atoms, optionally substituted by a hydroxy group, which is substituted at the terminal position by a phenyl or phenoxy group, whilst the phenyl nucleus is mono- or disubstituted in each case by alkyl and/or alkoxy groups with 1 to 3 carbon atoms each, and of the acid addition salts thereof, caracterised in that

a) a propoxyphenyl derivative of general formula

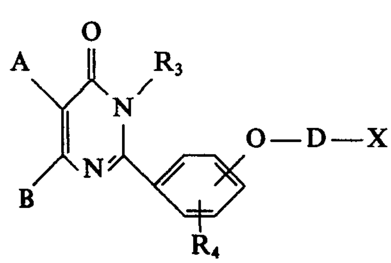

(II)

wherein
A, B, D, $R_3$ and $R_4$ are defined as mentioned before,
X represents a nucleophilically exchangeable group or
X together with the hydrogen atom in the $\beta$ position of the radical D represents an oxygen atom, is reacted with an amine of general formula

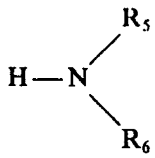

(III)

wherein

$R_5$ and $R_6$ are defined as mentioned before, or

b) a compound of general formula

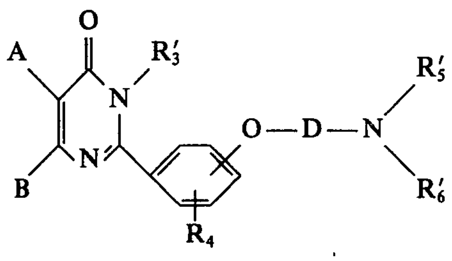

(IV)

wherein

A, B, D and $R_4$ are defined as mentioned before, at least one of the radicals $R'_3$, $R'_5$ or $R'_6$ represents a hydrogen atom and the remaining radicals $R'_3$, $R'_5$ or $R'_6$ have the meanings mentioned before for $R_3$, $R_5$ or $R_6$, is alkylated with a compound of general formula

$$Z—Y \qquad (V)$$

wherein

Z represents a nucleophilically exchangeable group or Z together with a hydrogen atom in the $\beta$ position of the radical $R_6$ represents an oxygen atom, and

Y has the meanings mentioned before for $R_3$,

$R_5$ and $R_6$, with the exception of a hydrogen atom, or with formaldehyde/formic acid, and subsequently, if desired a compound of general formula I thus obtained is converted into an acid addition salt thereof, especially a physiologically compatible acid addition salt thereof, with inorganic or organic acids.

2. Process as claimed in claim 1 for the preparation of new pyrimidinones of general formula

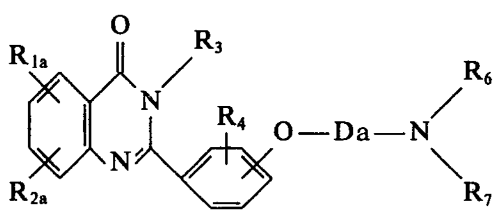

(Ia)

wherein

$R_3$, $R_4$, $R_6$ and $R_7$ are defined as in claim 1,

$R_{1a}$ represents a hydrogen or halogen atom, an amino or nitro group, or an alkyl or alkoxy group with 1 to 3 carbon atoms,

$R_{2a}$ represents a hydrogen atom or an alkoxy group with 1 to 3 carbon atoms and

Da represents an n-propylene group optionally substituted in the 2 position by a hydroxy group, and the acid additions salts thereof, characterised in that

a) a propoxyphenyl derivative of general formula

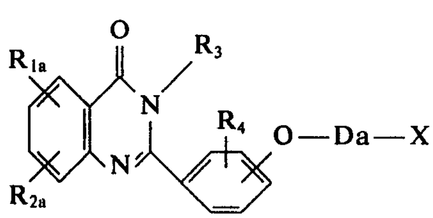

(IIa)

wherein

$R_{1a}$, $R_{2a}$, $R_3$, $R_4$ and Da are as hereinbefore defined,
X represents a nucleophilically exchangeable group or X together with the 2-hydroxy group represents an oxygen atom,
is reacted with an amine of general formula

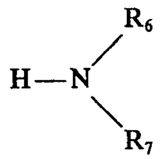

(III)

wherein
$R_6$ and $R_7$ are as hereinbefore defined, or

b) a compound of general formula

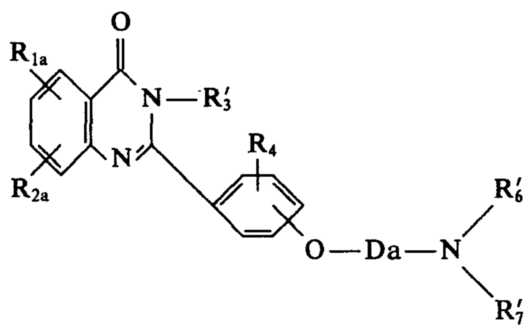

(IVa)

wherein
$R_{1a}$, $R_{2a}$, $R_4$ and Da are as hereinbefore defined and the groups $R_3'$, $R_6'$ and $R_7'$ have the meanings given in claim 1, is alkylated with a compound of general formula

$$Z—Y \quad (V)$$

wherein
Z and Y are defined as in claim 1,
or with formaldehyde/formic acid and subsequently, if desired, a compound of general formula I thus obtained is converted into an acid addition salt thereof, particularly a physiologically acceptable acid addition salt thereof, with inorganic or organic acids.
Priority: 12.12.1980 (DE-P 3046871.4)

3. Process as claimed in claim 1 for the preparation of new pyrimidinones of general formula

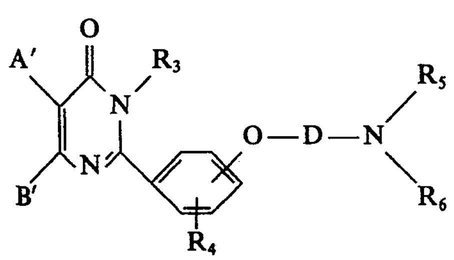

(Ib)

wherein
$R_3$ to $R_6$ and D are defined as in claim 1,
A′ and B′ together with the two carbon atoms between them represent a pyridine ring or else a methoxyphenyl ring if D represents a butylene group optionally substituted by a hydroxy group, or the propylene group, and the group

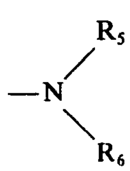

represents the 2-hydroxy-3-(4-methoxyphenoxy)-propylamino group, and of the acid addition salts thereof, characterised in that

a) a propoxyphenyl derivative of general formula

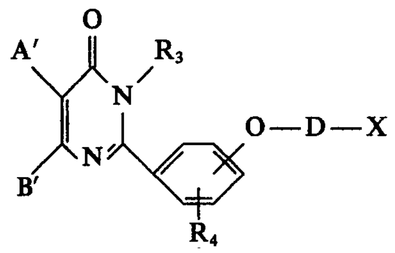

(IIb)

wherein
A′, B′, D, $R_3$ and $R_4$ are as hereinbefore defined and X is defined as in claim 1, is reacted with an amine of general formula

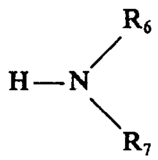

(III)

wherein
$R_6$ and $R_7$ are as hereinbefore defined, or

b) a compound of general formula

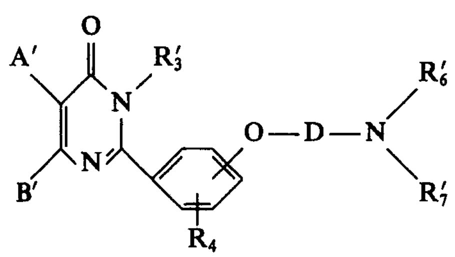

(IVb)

wherein
A′ and B′ are as hereinbefore defined and D, $R_4$, $R_3'$, $R_6'$ and $R_7'$ are defined as in claim 1, is alkylated with a compound of general formula

Z—Y (V)

wherein
Z and Y are defined as in claim 1, or with formaldehyde/formic acid
and subsequently, if desired, a compound of general formula I thus obtained is converted into an acid addition salt thereof, particularly a physiologically acceptable acid addition salt thereof, with inorganic or organic acids.
Priority: 16.04.1981 (DE-P 3 115 447.6)

4. A process as claimed in claims 1 a, 2 a and 3 a, characterised in that the reaction is carried out at temperatures between 50 and 200°C, but preferably at temperatures between 70 and 150°C.

5. A process as claimed in claims 1, 2, 3 and 4, characterised in that the reaction is carried out in a solvent.

6. A process as claimed in claims 1 a, 2 a, 3 a and 4, characterised in that the reaction is carried out in the presence of an acid-binding agent.

7. A process as claimed in claims 1 b, 2 b, 3 b and 5, characterised in that the reaction is carried out at temperatures between 0 and 180°C, but preferably at the boiling temperature of the reaction mixture.

8. A process as claimed in claims 1 b, 2 b, 3 b, 5 and 7, characterised in that the reaction is carried out in the presence of an inorganic or tertiary organic base.

**Revendications pour les Etats contractants: BE, CH, DE, FR, IT, LI, LU, NL, SE.**

1. Nouvelles pyrimidinones de formule générale:

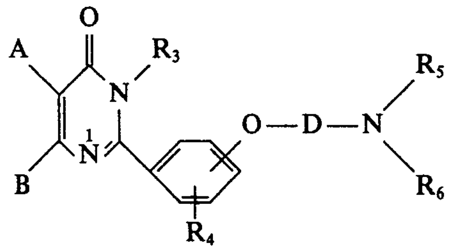

(I)

dans laquelle:

A et B représentent ensemble avec les deux atomes de carbone situés entre eux cycle pyridine ou un cycle phényle qui peut être substitué par les radicaux $R_1$ et/ou $R_2$, où

$R_1$ représente un atome d'halogène, un groupe amino ou nitro, un groupe alcoyle ou alcoxy avec chacun 1 à 3 atomes de carbone, et
$R_2$ représente un groupe alcoxy avec 1 à 3 atomes de carbone,

D représente un groupe alcoylène avec 3 ou 4 atomes de carbone éventuellement substitué par un groupe hydroxy,
$R_3$ et $R_5$, qui peuvent être identiques ou différents, représentent chacun un atome d'hydrogène ou un groupe alcoyle avec 1 à 3 atomes de carbone,
$R_4$ représente un atome d'hydrogène ou un groupe alcoxy avec 1 à 3 atomes de carbone, et
$R_6$ représente un groupe alcoyle rectiligne ou ramifié avec 1 à 6 atomes de carbone ou un groupe alcoylène rectiligne, saturé avec 2 à 4 atomes de carbone éventuellement substitué par un groupe hydroxy, lequel groupe alcoylène est substitué à son extrémité par un groupe phényle ou phénoxy, le noyau phényle pouvant être mono- ou disubstitué à chaque fois par des groupes alcoyle et/ou alcoxy avec chacun 1 à 3 atomes de carbone, et leurs sels d'addition d'acides.

2. Nouvelles pyrimidinones de formule générale I selon la revendication 1, dans laquelle:

A et B représentent ensemble avec les deux atomes de carbone situés entre eux un cycle pyridine ou un cycle phényle qui peut être substitué par les radicaux $R_1$ et/ou $R_2$, où $R_1$ représente un atome de chlore, un groupe méthyle, méthoxy ou nitro et
$R_2$ représente un groupe méthoxy,
D représente un groupe n-propylène, n-butylène, 2-hydroxy-n-propylène, 2-hydroxy-n-butylène ou 3-hydroxy-n-butylène,
$R_3$ et $R_5$ qui peuvent être identiques ou différents, représentent chacun un atome d'hydrogène ou un groupe alcoyle avec 1 à 3 atomes de carbone,
$R_4$ représente un atome d'hydrogène ou le groupe méthoxy et
$R_6$ représente un groupe alcoyle avec 1 à 4 atomes de carbone, un groupe éthyle substitué en position 2 par un groupe méthoxyphényle, diméthoxyphényle, méthylphénoxy ou méthoxyphénoxy ou représente un groupe 2-hydroxypropyle substitué en position 3 par un groupe méthoxyphénoxy ou méthylphénoxy, et leurs sels d'addition d'acides, en particulier leurs sels d'addition d'acides physiologiquement supportables avec des acides minéraux ou organiques.

3. Nouvelles pyrimidinones de formule générale I selon la revendication 1, dans laquelle:

A et B représentent ensemble avec les deux atomes de carbone situés entre eux un cycle phényle, méthoxyphényle, diméthoxyphényle ou pyridine,
D représente le groupe n-propylène ou 2-hydroxy-n-propylène,
$R_3$ représente un atome d'hydrogène ou le groupe méthyle,
$R_4$ représente un atome d'hydrogène ou le groupe méthoxy,
$R_5$ représente un atome d'hydrogène ou un groupe alcoyle avec 1 à 3 atomes de carbone et
$R_6$ représente un groupe alcoyle avec 1 à 4 atomes de carbone, un groupe éthyle substitué en position 2 par un groupe méthoxyphényle, diméthoxyphényle, méthylphénoxy ou méthoxyphénoxy ou représente un groupe 2-hydroxypropyle substitué en position 3 par un groupe méthoxyphénoxy ou méthylphénoxy, et leurs sels d'addition d'acides physiologiquement avec des acides minéraux ou organiques.

4. Nouvelles pyrimidinones de formule générale:

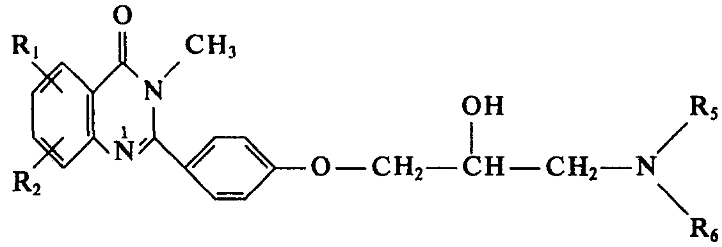

(Ia)

dans laquelle:

$R_1$ représente un groupe méthoxy en position 6 ou 8,
$R_2$ représente un atome d'hydrogène ou en position 7 un groupe méthoxy et
$R_5$ et $R_6$ représentent ensemble avec l'atome d'azote situé entre eux un groupe isopropylamino, tert.-butylamino, N-méthyl-2-(3,4-diméthoxyphényl)-éthylamino, 2-(2-méthoxyphényl)-éthylamino, 2-(2-méthylphénoxy)-éthylamino ou 2-(4-méthoxyphénoxy)-éthylamino et leurs sels d'addition d'acides physiologiquement supportables avec des acides minéraux ou organiques.

5. La 2-[4-(2-hydroxy-3-tert.butylamino-propoxy)-phényl]-3-méthyl-6-méthoxy-3,4-dihydro-quinazoline-4-one et sels d'addition d'acides.

6. La 2-[4-[2-hydroxy-3-(2-(3,4-diméthoxyphényl)-N-méthyl-éthylamino)-propoxy]-phényl]-3-méthyl-6-méthoxy-3,4-dihydro-quinazoline-4-one et ses sels d'addition d'acides.

7. La 2-[4-[2-hydroxy-3-(2-(2-méthoxyphényl)-éthylamino)-propoxy]-phényl]-3-méthyl-6-méthoxy-3,4-dihydro-quinazoline-4-one et ses sels d'addition d'acides.

8. Médicament contenant un composé selon les revendications 1 à 7 conjointement à un ou plusieurs excipients et/ou diluants inertes.

9. Utilisation d'un composé selon les revendications 1 à 7 pour la préparation d'un médicament par voie non chimique pour lutter contre l'hypertension et les maladies des coronaires.

10. Procédé pour la préparation de nouvelles pyrimidinones de formule générale:

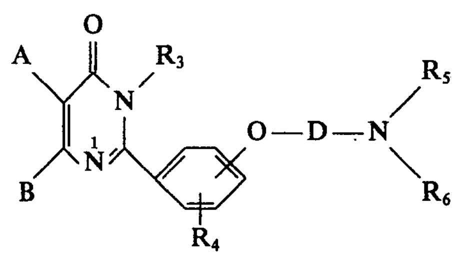

(I)

dans laquelle:

A et B représentent ensemble avec les deux atomes de carbone situés entre eux un cycle pyridine ou un cycle phényle, qui peut être substitué par les radicaux $R_1$ et/ou $R_2$, où

- $R_1$ représente un atome d'halogène, un groupe amino ou nitro, un groupe alcoyle ou alcoxy avec chacun 1 à 3 atomes de carbone, et
- $R_2$ représente un groupe alcoxy avec 1 à 3 atomes de carbone,

D représente un groupe alcoylène avec 3 ou 4 atomes de carbone éventuellement substitué par un groupe hydroxy,
$R_3$ et $R_5$, qui peuvent être identiques ou différents, représentent chacun un atome d'hydrogène ou un groupe alcoyle avec 1 à 3 atomes de carbone,
$R_4$ représente un atome d'hydrogène ou un groupe alcoxy avec 1 à 3 atomes de carbone et
$R_6$ représente un groupe alcoyle rectiligne ou ramifié avec 1 à 6 atomes de carbone ou un groupe alcoylène rectiligne, saturé avec 2 à 4 atomes de carbone éventuellement substitué par un groupe hydroxy, lequel groupe alcoylène est substitué à son extrémité par un groupe phényle ou phénoxy, le noyau phényle pouvant être mono- ou disubstitué dans chaque cas par des groupes alcoyle et/ou alcoxy avec chacun 1 à 3 atomes de carbone, et de leurs sels d'addition d'acides caractérisé en ce que:

a) on fait réagir un dérivé propoxyphénylé de formule générale

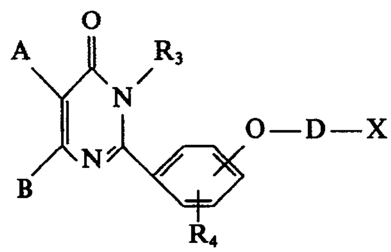

(II)

dans laquelle:
A, B, D, $R_3$ et $R_4$ sont définis comme au début,
X représente un groupe échangeable de façon nucléophile ou X représente ensemble avec l'atome d'hydrogène se trouvant en position $\beta$ du radical D un atome d'oxygène, avec une amine de formule générale:

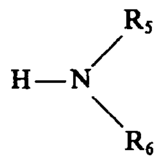

(III)

dans laquelle:
$R_5$ et $R_6$ sont définis comme au début, ou

b) on alcoyle un composé de formule générale

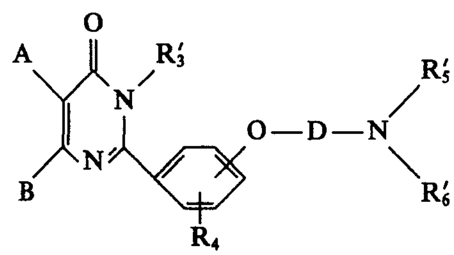

(IV)

dans laquelle:
A, B, D et $R_4$ sont définis comme au début,
au moins l'un des radicaux $R'_3$, $R'_5$ ou $R'_6$ représente un atome d'hydrogène et les autres des radicaux $R'_3$, $R'_5$ ou $R'_6$ possèdent les significations mentionnées au début pour $R_3$, $R_5$ ou $R_6$, au moyen d'un composé de formule générale:

$$Z—Y \qquad (V)$$

dans laquelle:
Z représente un groupe échangeable de façon nucléophile ou Z représente ensemble avec un atome d'hydrogène se trouvant en position $\beta$ du radical $R_6$ un atome d'oxygène et Y possède, à l'exception de celle d'un atome d'hydrogène, les significations mentionnées au début pour $R_3$, $R_5$ et $R_6$, ou au moyen de formaldéhyde/acide formique et si on le désire ensuite, on transforme un composé ainsi obtenu de formule générale I en son sel d'addition d'acide, en particulier en son sel d'addition d'acide physiologiquement supportable avec des acides minéraux ou organiques.

**Revendications pour l'Etat contractant: AT**

1. Procédé pour la préparation de nouvelles pyrimidinones de formule générale:

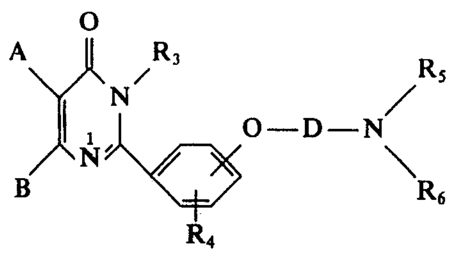

(I)

dans laquelle:

A et B représentent ensemble avec les deux atomes de carbone situés entre eux cycle pyridine ou un cycle phényle qui peut être substitué par les radicaux $R_1$ et/ou $R_2$, où

$R_1$ représente un atome d'halogène, un groupe amino ou nitro, un groupe alcoyle ou alcoxy avec chacun 1 à 3 atomes de carbone, et

$R_2$ représente un groupe alcoxy avec 1 à 3 atomes de carbone,

D représente un groupe alcoylène avec 3 ou 4 atomes de carbone éventuellement substitué par un groupe hydroxy,

$R_3$ et $R_5$, qui peuvent être identiques ou différents, représentent chacun un atome d'hydrogène ou un groupe alcoyle avec 1 à 3 atomes de carbone,

$R_4$ représente un atome d'hydrogène ou un groupe alcoxy avec 1 à 3 atomes de carbone, et

$R_6$ représente un groupe alcoyle rectiligne ou ramifié avec 1 à 6 atomes de carbone ou un groupe alcoylène rectiligne, saturé avec 2 à 4 atomes de carbone éventuellement substitué par un groupe hydroxy, lequel groupe alcoylène est substitué à son extrémité par un groupe phényle ou phénoxy, le noyau phényle pouvant être mono- ou disubstitué à chaque fois par des groupes alcoyle et/ou alcoxy avec chacun 1 à 3 atomes de carbone et de leurs sels d'addition d'acides, caractérisé en ce que:

a) on fait réagir un dérivé propoxyphénylé de formule générale

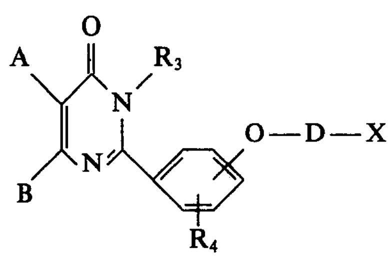

(II)

dans laquelle:
A, B, D, $R_3$ et $R_4$ sont définis comme au début,
X représente un groupe échangeable de façon nucléophile ou X représente ensemble avec un atome d'hydrogène se trouvant en position $\beta$ du radical D un atome d'oxygène, avec une amine de formule générale:

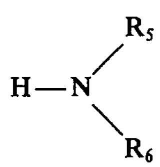

(III)

dans laquelle:
$R_5$ et $R_6$ sont définis comme au début, ou

b) on alcoyle un composé de formule générale

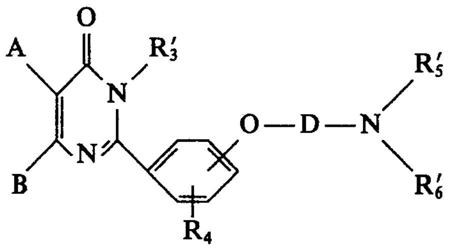

(IV)

dans laquelle:
A, B, D et $R_4$ sont définis comme au début,
au moins l'un des radicaux $R'_3$, $R'_5$ ou $R'_6$ représente un atome d'hydrogène et les autres des radicaux $R'_3$, $R'_5$ ou $R'_6$ possèdent les significations mentionnées au début pour $R_3$, $R_5$ ou $R_6$, au moyen d'un composé de formule générale:

$$Z—Y \qquad (V)$$

dans laquelle:
Z représente un groupe échangeable de façon nucléophile ou Z représente ensemble avec un atome d'hydrogène se trouvant en position $\beta$ du radical $R_6$ un atome d'oxygène et
Y possède, à l'exception de celle d'un atome d'hydrogène, les significations mentionnées au début pour $R_3$, $R_5$ et $R_6$, ou au moyen de formaldéhyde/acide formique et si on le désire ensuite, on transforme un composé ainsi obtenu de formule générale I en son sel d'addition d'acide, en particulier en son sel physiologiquement supportable, avec des acides minéraux ou organiques.

2. Procédé selon la revendication 1 pour la préparation de nouvelles pyrimidinones de formule générale:

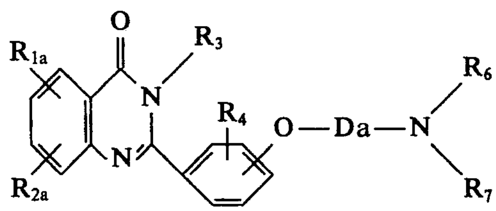

(Ia)

dans laquelle:
$R_3$, $R_4$, $R_6$ et $R_7$ sont définis comme dans la revendication 1,
$R_{1a}$ représente un atome d'hydrogène ou d'halogène, un groupe amino ou nitro, un groupe alcoyle ou alcoxy avec chacun 1 à 3 atomes de carbone,
$R_{2a}$ représente un atome d'hydrogène ou un groupe alcoxy avec 1 à 3 atomes de carbone et
Da représente un groupe n-propylène éventuellement substitué en position 2 par un groupe hydroxy et de leurs sels d'addition d'acides, caractérisé en ce que:

a) on fait réagir un dérivé propoxyphénylé de formule générale

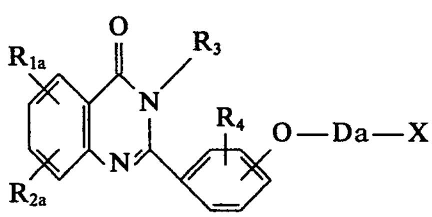

(IIa)

dans laquelle:
$R_{1a}$, $R_{2a}$, $R_3$, $R_4$ et Da sont définis comme au début,
X représente un groupe échangeable de façon nucléophile ou X représente ensemble avec le groupe 2-hydroxy un atome d'oxygène, avec une amine de formule générale:

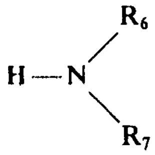

(III)

dans laquelle:
$R_6$ et $R_7$ sont définis comme au début ou

b) on alcoyle un composé de formule générale

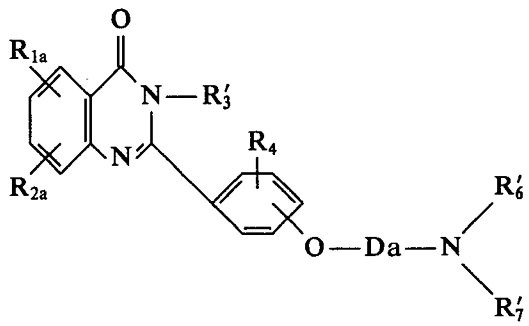

(IVa)

dans laquelle:
$R_{1a}$, $R_{2a}$, $R_4$ et Da sont définis comme au début et les radicaux $R'_3$, $R'_6$ et $R'_7$ possèdent les significations mentionnées dans la revendication 1, au moyen d'un composé de formule générale:

Z—Y (V)

dans laquelle:
Z et Y sont définis comme dans la revendication 1, ou au moyen de formaldéhyde/acide formique et en ce que si on le désire ensuite on transforme un composé ainsi obtenu de formule générale I en son sel d'addition d'acide, en particulier en son sel d'addition d'acide physiologiquement supportable, avec des acides minéraux ou organiques.
Priorité: 12.12.1980 (demande de brevet DE 3 046 871.4).

3. Procédé selon la revendication 1 pour la préparation de nouvelles pyrimidinones de formule générale:

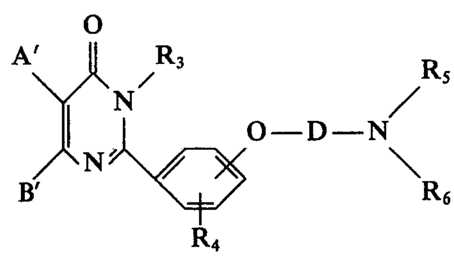

(Ib)

dans laquelle:
$R_3$ à $R_6$ et D sont définis comme dans la revendication 1,
A' et B' représentent ensemble avec les deux atomes de carbone situés entre eux un cycle pyridine ou également un cycle méthoxyphényle, lorsque D représente un groupe butylène éventuellement substitué par un groupe hydroxy ou le groupe propylène et le groupe

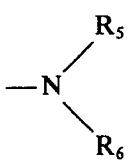

représente le groupe 2-hydroxy-3-(4-méthoxyphénoxy)-propylamino, et de leurs sels d'addition d'acides caractérisé en ce que:

a) on fait réagir un dérivé propoxyphénylé de formule générale

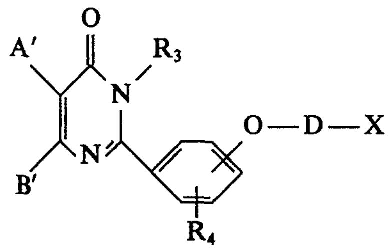

(IIb)

dans laquelle:
A′, B′, D, $R_3$ et $R_4$ sont définis comme au début, et X est défini comme dans la revendication 1, avec une amine de formule générale:

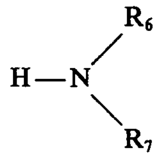

(III)

dans laquelle:
$R_6$ et $R_7$ sont définis comme au début, ou

b) on alcoyle un composé de formule générale

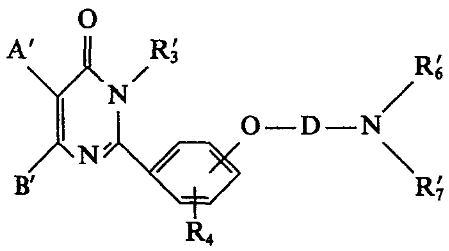

(IVb)

dans laquelle:
A′ et B′ sont définis comme au début et D, $R_4$, $R'_3$, $R'_6$ et $R'_7$ sont définis comme dans la revendication 1, au moyen d'un composé de formule générale:

Z—Y (V)

dans laquelle:
Z et Y sont définis comme dans la revendication 1, ou au moyen de formaldéhyde/acide formique et si on le désire ensuite on transforme un composé obtenu de formule générale I en son sel d'addition d'acide, en particulier en son sel physiologiquement supportable, avec des acides minéraux ou organiques.
Priorité: 16.04.1981 (demande de brevet DE 3 115 447.6).

4. Procédé selon les revendications 1 a, 2 a et 3 a, caractérisé en ce que la réaction est effectuée à des températures entre 50 et 200°C, de préférence cependant à des températures entre 70 et 150°C.

5. Procédé selon les revendications 1, 2, 3 et 4, caractérisé en ce que la réaction est effectuée dans un solvant.

6. Procédé selon les revendications 1 a, 2 a, 3 a et 4, caractérisé en ce que la réaction est effectuée en présence d'un agent fixant les acides.

7. Procédé selon les revendications 1 b, 2 b, 3 b et 5, caractérisé en ce que la réaction est effectuée à des températures entre 0 et 180°C, de préférence cependant à la température d'ébullition du mélange réactionnel.

8. Procédé selon les revendications 1 b, 2 b, 3 b, 5 et 7, caractérisé en ce que la réaction est effectuée en présence d'une base minérale ou organique tertiaire.